(19) Europäisches Patentamt / European Patent Office / Office européen des brevets

(11) **EP 3 705 890 A1**

(12) **EUROPEAN PATENT APPLICATION**

(43) Date of publication:
**09.09.2020 Bulletin 2020/37**

(51) Int Cl.:
***G01N 33/50*** *(2006.01)*

(21) Application number: **19305262.8**

(22) Date of filing: **06.03.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
• **Institut Regional du Cancer de Montpellier**
**34090 Montpellier (FR)**

• **Université de Montpellier**
**34090 Montpellier (FR)**

(72) Inventors:
• **AZRIA, David**
**30250 FONTANES (FR)**
• **GOURGOU, Sophie**
**34830 JACOU (FR)**

(74) Representative: **Flesselles, Bruno F.G.**
**BF IP**
**36 rue Jean de la Fontaine**
**75016 Paris (FR)**

(54) **IN VITRO METHOD FOR ASSESSING THE RISK OF PROSTATE SIDE EFFECT AFTER TREATMENT BY IONIZING RADIATION**

(57) The present invention relates to an *in vitro* method for assessing the risk of developing side effects after ionizing radiation treatment in a prostate cancer subject, comprising the steps of

a) measuring radiation induced T-lymphocytes apoptosis in a sample of the subject;

b) determining the presence of urinary toxicity in the patient prior to application of ionizing radiation, and optionally of at least one other clinical parameter, disease parameter or ionizing radiation treatment parameter from the patient, and

c) combining the value of the at least one biochemical marker measured in step (a) and a value associated with the at least one clinical parameter, disease parameter or ionizing radiation treatment parameter determined in step (b) in a mathematical function to obtain an end-value.

**Description**

**FIELD OF INVENTION**

The present invention relates to the prediction of a risk of developing side effects after treatment by ionizing radiation (TIR) in a prostate cancer patient. The present invention thus provides an *in vitro* method and a calculator for assessing the risk of developing side effects after TIR.

**BACKGROUND OF INVENTION**

Treatment by ionizing radiation (TIR) is one of the leading treatment modalities in oncology, and over 50% of patients diagnosed with cancer undergo TIR during their course of treatment. Also TIR is primarily a local treatment, patients are exposed to a risk of toxicities in the treatment field and surrounding tissues, which may develop acutely (*i.e.*, in the first 3 months) or late (*i.e.*, more than 3 months following RT). Severe acute toxicities may have late consequences as recovering may be incomplete. In addition, late toxicities may occur over time and often persist with significant negative impact on quality of life among cancer survivors.

A number of factors are known to increase the risk of radiation-induced toxicity, including individual radiosensitivity (Azria, Betz et al. 2012). While toxicity risks for populations of patients are known, the determination of an individual's normal tissue radiosensitivity is seldom possible before treatment. Therefore, current practice standards commonly prescribe radiation dose according to clinical scenarios from standard recommendations, without regard to the genotype or phenotype of the individual being irradiated.

In that context, a radiosensitivity prognostic test was developed, based on flow cytometric assessment of RILA (radiation-induced T-lymphocytes apoptosis). This prognostic test was described as having a clear potential for selecting individuals likely to display an increased probability of toxicity to TIR (Ozsahin, Crompton et al. 2005). Nevertheless, the prediction of this radiosensitivity assay based on RILA exhibits some limitations in terms of sensitivity and reliability. Indeed, taken alone, RILA is not capable of exhaustive prediction in terms of sensitivity and specificity.

Therefore, there is an important need to develop a method of prognostic predicting the risk of developing radiation-induced side effects, such as prostate side effects after TIR, having an enhanced sensitivity and reliability.

Foro et al (Int J Radiat Oncol Biol Phys. 2014 Apr 1;88(5):1057-63) assess the correlation between radiation-induced apoptosis of T lymphocytes (RILA) and chronic toxicity in prostate cancer patients. They propose to use univariate or multivariate Cox proportional hazard models. Tables 3 and 4 present various parameters proposed by the authors. These parameters don't include the presence of urinary toxicity before treatment.

De Langhe et al. (2013, Radiotherapy and Oncology, Volume 106, S350) present an abstract pertaining to the prediction of occurrence of radiation-induced genitourinary (GU) symptoms such as nocturia or hematuria. They combine multiple factors including clinical, treatment, dosimetric and genetic parameters. Presence of pre-treatment urinary disorders or RILA are not among these parameters.

US 2016/008629 discloses a system and method for detecting or predicting toxicity induced by radiation therapy of prostate cancer, in particular by determining polypeptide biomarkers present in a urine sample. It proposes combine the biomarker concentrations with data from, for example, medical imaging, disease and/or patient specific information, such as genetic data, tumor grade and stage, and co-morbidities to obtain even more reliable predictions. However, no such combination is provided, and RILA or presence of pre-treatment urinary disorders are not cited.

WO2018/041960 discloses a mathematical function combining RILA and other factors (such as tabagism or presence of concomitant hormonotherapy) to detect the occurrence of late effects in breast cancer patient treated with radiotherapy. The factors combined with RILA are adapted for breast cancer but not for prostate cancer.

**SUMMARY**

The invention now provides a new prognosis method for predicting the probability or risk of developing radiation-induced side effects, in particular a prostate side effect (PSE), wherein RILA is combined with the presence of pre-treatment urinary toxicity, and optionally with other clinical parameters, disease parameters and/or ionizing radiation treatment parameters, and in particular with the stade of the tumor (according to TNM classification), neoadjuvant, concomitant and/or adjuvant hormonotherapy, with an improved global evaluation of the risk of developing a PSE for a patient. This method is preferably performed before treatment of the patient. This method makes it possible to make a prognosis of the occurrence of such PSE, in particular within two years (24 months) after the ionizing radiation treatment. Full advantages of the method are to be found when it is performed before the start of treatment of a patient with prostate cancer, as it provides the physician with valuable information as to the nature of the treatment that can be proposed to the patient, depending on the patient's risk of having some PSE.

The inventors show herein that RILA alone is not sufficient to provide prognosis of occurrence of side effect after radiation treatment of prostate cancer, and that it shall be combined with other parameters, among which presence (and optionally grade) of urinary disorders. These two parameters are preferably combined with at least one other parameter, in particular with exactly one other parameter, or with two other parameters, or with three other parameters, or with four other parameters. It is also possible to use further parameters. It is believed that it is the first time that a link between presence of urinary disorders prior to treatment and susceptibility to the occurrence of side effects has been shown in prostate cancer patients.

The present invention relates to an *in vitro* method for assessing the risk of developing side effects after ionizing radiation in a patient suffering from prostate cancer, comprising:

a) measuring at least one biochemical marker in a sample of the patient, wherein said at least one biochemical marker is radiation induced T-lymphocytes apoptosis (RILA);

b) determining the presence of urinary toxicity in the patient, and optionally of at least one other clinical parameter, disease parameter or ionizing radiation treatment parameter from the patient, wherein said at least one clinical parameter, disease parameter or ionizing radiation comprises presence of urinary toxicity prior to application of ionizing radiation, and

c) combining the value of the at least one biochemical marker measured in step (a) a value associated with the presence of urinary toxicity, and optionally values associated with the at least one other clinical parameter, disease parameter or ionizing radiation treatment parameter determined in step (b) in a mathematical function to obtain an end-value.

Such end-value can later be compared to a reference value, in order to determine the risk, for the patient to developing side effects after a ratio-therapy treatment, or a treatment with ionizing radiation. This method thus also makes it possible to determine if the patient a no, or a low risk to develop such side effects.

This method is preferably performed before starting the treatment of the patient. In this case, if a ionizing radiation treatment parameter is used in the method, the ionizing radiation treatment parameter is the one that is envisaged for the patient.

In one embodiment, the radiation induced T-lymphocytes apoptosis (RILA) measured in a) is radiation induced CD8$^+$T-lymphocytes apoptosis.

In one embodiment, the method comprises measuring or determining at least one other clinical marker than urinary toxicity, preferably selected from the group consisting of the presence or absence of concomitant and/or adjuvant hormonotherapy, the tobacco smoking habit, the presence of gastrointestinal toxicity, the dosage of PSA (Prostate Specific Antigen), the tumor T stage (according to TNM classification), the tumor N stage, and the presence of at least one comorbidity. In this case, a value associated with said at least one other clinical marker is also used and combined in the function of c). If more than one other clinical marker is also measured or determined, the values associated with each of the measured or determined clinical markers are used in c).

In one embodiment, the age of the patient is also taken into consideration and, combined in the function of c)..

In one embodiment, the method comprises using at least one ionizing radiation treatment parameter, preferably CTV, CTV1 and/or CTV2 in b) for combination in c).

In one embodiment, the mathematical function of c) is a multivariate analysis using a binary logistic regression, a multiple linear regression or any time dependent regression a binary logistic regression, a multiple linear regression or any time dependent regression.

In one embodiment, said mathematical function is a Cox proportional hazard regression model.

In one embodiment, the method of the invention further comprises a step of comparing the end-value with a reference end-value, thereby determining if the subject present a high or low risk to develop radiation-induced side-effects. This risk may be a relative risk (i.e. as compared to the mean risk in the population).

In one embodiment, the sample is a T cell containing sample, preferably a blood sample.

In one embodiment, said radiation-induced side effect is a side effect occurring during ionizing radiation and/or during the follow-up after ionizing radiation, such as, for example, 1 month, 3, 6, 12, 18, 24, 30 or 36 months after the end of the ionizing radiation. In particular, the method is particularly adapted to determine the risk of occurence of a PSE (radiation-induced side effect) in the 24 months following the treatment.

In one embodiment, said radiation-induced side effect is selected from urinary toxicities, gastrointestinal toxicities, sexual toxicities and pelvic neuropathy.

In one embodiment, said method is computerized.

The present invention further relates to a microprocessor comprising a software for implementing the *in vitro* method as described hereinabove.

The present invention also relates to a method for treating a patient having prostate cancer, comprising:

a) performing a method as disclosed above, and

b) treating the patient with ionizing radiation, if the end result indicates that the subject has no or a low risk of developing side effects after ionizing radiation,

c) providing another treatment or adapting the ionizing radiation treatment if the patient doesn't have no or a low risk of developing side effects after ionizing radiation.

In particular, the ionizing radiation treatment can be adapted taking into account the risk of developing PSE together with the risk of tumor recurrence.

The invention also relates to a method for obtaining a mathematical function that can be used in an in vitro non-invasive diagnosis test for assessing the risk of developing side effects after ionizing radiation in a subject suffering from prostate cancer, comprising:

a) classifying subjects of a cohort of patients into different groups according to the presence of side effects after ionizing radiation

b) providing the value at least one biochemical marker in a sample of each subjects, wherein said at least one of biochemical marker is radiation induced T-lymphocytes apoptosis (RILA) measured before ionizing radiation treatment; and

c) determining the presence or absence of at least one clinical parameter, disease parameter from each patient, wherein said at least one clinical parameter, disease parameter comprises presence of urinary toxicity prior to ionizing radiation treatment

d) optionally determining the presence of at least one other marker selected from the group consisting in the presence or absence of concomitant and/or adjuvant hormonotherapy, the tobacco smoking habit, the presence of gastrointestinal toxicity, the dosage of PSA (Prostate Specific Antigen), the tumor T stage, the tumor N stage, the presence of at least one comorbidity (in particular diabetes) and age of the patient, before ionizing radiation treatment

e) identifying by unidimensional analysis, the markers measured in step d) for which the measured value differs significantly between the group of subjects in which side effects after ionizing radiation have developed and the group of subjects in which side effects after ionizing radiation have not occurred, thereby obtaining independent factors

f) obtaining the mathematical function by combination of the factor measured in b), the marker determined in c) and the identified independent factors identified in e).

## DEFINITIONS

In the present invention, the following terms have the following meanings:

- The terms "**a**" and "**an**" refer to one or to more than one (*i.e.*, to at least one) of the grammatical object of the article. By way of example, "an element" means one element or more than one element.
- The term "**about**" when referring to a measurable value such as an amount, a temporal duration, and the like, is meant to encompass variations of $\pm 20\%$ or in some instances $\pm 10\%$, or in some instances $\pm 5\%$, or in some instances $\pm 1\%$, or in some instances $\pm 0.1\%$ from the specified value, as such variations are appropriate to perform the disclosed methods.
- The term "**biochemical marker**" refers to a variable that may be measured in a sample from the subject, said sample being preferably a blood sample.
- The term "**Cox regression**" refers to a usual statistical model for time-to-event analysis (Cox, et al. 1984). Apart from a classification algorithm which directly deals with binary or multi-class outcomes, Cox regression defines a semi-parametric model to directly relate the predictive variables with the real outcome, which may be, for example, a survival time (*e.g.*, in months or years) or a time without occurrence of side effects or recurrence of a disease. Multivariate Cox function is considered as the best hazard function in terms of discrimination for time-to-event endpoint to combine independent parameters.
- The term "**encoding**" refers to the inherent property of specific sequences of nucleotides in a polynucleotide, such as a gene, a cDNA, or an mRNA, to serve as templates for synthesis of other polymers and macromolecules in biological processes having either a defined sequence of nucleotides (*e.g.*, rRNA, tRNA and mRNA) or a defined sequence of amino acids and the biological properties resulting therefrom. Thus, a gene, cDNA, or RNA, encodes a protein if transcription and translation of mRNA corresponding to that gene, or cDNA, produces the protein in a cell or other biological system. Both the coding strand, the nucleotide sequence of which is identical to the mRNA sequence and is usually provided in sequence listings, and the non-coding strand, used as the template for transcription of a gene or cDNA, can be referred to as encoding the protein or other product of that gene or cDNA.
- The term "**expression**" refers to the transcription and/or translation of a particular nucleotide sequence driven by a promoter.

- The term "**instructional material**" includes a publication, a recording, a diagram, or any other medium of expression which can be used to communicate the usefulness of the kit of the invention. The instructional material of the kit of the invention may, for example, be affixed to a container which contains the reagents for implementing the method of the invention or be shipped together with a container which contains the reagents for implementing the method of the invention. Alternatively, the instructional material may be shipped separately from the container with the intention that the instructional material be used cooperatively by the recipient.

- The term "*in vitro* **method**" refers to a method comprising steps performed *in vitro* (*e.g.*, a RILA assay) or *ex-vivo* (*e.g.*, multivariate cox regression model obtained with clinical parameters or disease parameters or ionizing radiation treatment parameters previously evaluated on patients).

- The term "**non-invasive**", when referring to a method according to the present invention, means that the method of the invention does not comprise obtaining a tissue sample from the body of a subject. In one embodiment, a blood sample is not considered as a tissue sample.

- "**ROC**" In statistics, a receiver operating characteristic (ROC), or ROC curve, is a graphical plot that illustrates the performance of a binary classifier system as its discrimination threshold is varied. The curve is created by plotting the sensitivity against the specificity (usually 1- specificity) at successive values from 0 to 1. "**AUROC**" stands for area under the ROC curve, and is an indicator of the accuracy of a prognostic or diagnostic test. ROC curve and AUROC are well-known in the field of statistics.

- The term "**sensitivity of a method of prognosis**" refers to the proportion of patients with a risk to develop side-effect that are correctly identified as such using a method of prognosis.

- The term "**specificity of a method of prognosis**" refers to a measure of the proportion of patients without risk to develop side-effect that are correctly identified as such using a method of prognosis.

- The term "**side effect**" (or adverse event) refers to an unfavorable and unintended sign (including an abnormal laboratory finding), symptom or disease temporally associated with the use of a medical treatment. In particular, a radiation-induced side effect is a side effect induced in a subject by an ionizing radiation treatment. Severity of side effects may be defined according to the Common Terminology Criteria for Adverse Events (CTCAE, e.g., CTCAE v3.0 or CTCAE v4.0). According to the CTCAE, globally the following grades of side effects may be distinguished: Grade 1: mild side effect, Grade 2: moderate side effect, Grade 3: severe side effect, Grade 4: life threatening or disabling side effect and Grade 5: death related to side effect, but specifically defined for each symptom. In one embodiment of the invention, the side effect is at least a Grade 2 side effect. In one embodiment, the side effect is a Grade 2, 3, 4 or 5 side-effect, preferably a Grade 2, 3 or 4 side-effect. In the present description, reference is made to the CTCAE V3.0, published by the U.S.DEPARTMENT OF HEALTH AND HUMAN SERVICES (National Institutes of Health, National Cancer Institut, Cancer Therapy Evaluation Program), March 31, 2003 and published on August 9, 2006.

- The term "**subject**" or "**patient**" is intended to include any living organisms (*e.g.*, mammals, preferably humans). In one embodiment, a subject is a warm-blooded animal, preferably a human, who/which is awaiting the receipt of, or is receiving medical care or was/is/will be the object of a medical procedure, or is monitored for the development of the targeted disease or condition. In one embodiment, the subject is an adult (for example a subject above the age of 18). In another embodiment, the subject is a child (for example a subject below the age of 18).

- The terms "**treat**", "**treatment**" and "**treating**" refer to the reduction or amelioration of the progression, severity and/or duration of a targeted cancer, or to the amelioration of one or more symptoms (preferably, one or more discernible symptoms) of a targeted cancer, wherein said amelioration results from the administration of one or more therapies. In one embodiment the terms "treat", "treatment" and "treating" refer to the inhibition of the progression of a targeted cancer, either physically by, e.g., stabilization of at least one discernible symptom, physiologically by, e.g., stabilization of a physical parameter, or both. In other embodiments the terms "treat", "treatment" and "treating" refer to the reduction or amelioration of the progression, severity and/or duration of a targeted cancer, or to the amelioration of one or more symptoms of a targeted cancer. A subject is successfully "treated" for a cancer if, after receiving a therapeutically effective amount of a therapeutic agent, the subject shows observable and/or measurable reduction in the number of pathogenic cells or reduction in the percent of total cells that are pathogenic; relief to some extent of one or more of the symptoms associated with the specific cancer; reduced morbidity and mortality, and/or improvement in quality of life issues. The above parameters for assessing successful treatment and improvement in the condition are readily measurable by routine procedures familiar to a physician.

## DETAILED DESCRIPTION

The present invention first relates to an *in vitro* method for assessing the risk of developing side effects after ionizing radiation in a subject having prostate cancer, wherein said method comprises the steps of:

a) measuring the rate of Radiation-Induced Lymphocytes Apoptosis in a sample of the subject and optionally at least one other biochemical marker;
b) determining the presence or absence a urinary toxicity in the subject,
c) Optionally measuring or determining for the subject at least one other of:

- at least one clinical parameter; and/or
- at least one disease parameter; and/or
- at least one ionizing radiation treatment parameter, and

d) combining

- the value of the at least one biochemical marker measured in step (a)
- a value associated with the presence or absence of urinary toxicity, and
- the values associated with any orther clinical, disease or ionizing radiation treatment parameter of c), if relevant,

in a mathematical function to obtain an end-value.

In one embodiment, the method of the invention comprises measuring or determining at least one other clinical parameter. In one embodiment, the method of the invention comprises measuring or determining at least one disease parameter. In one embodiment, the method of the invention comprises measuring or determining at least one ionizing radiation treatment parameter. In one embodiment, the method of the invention comprises measuring or determining at least one other clinical parameter and at least one disease parameter. In one embodiment, the method of the invention comprises measuring or determining at least one other clinical parameter and at least one ionizing radiation treatment parameter. In one embodiment, the method of the invention comprises measuring or determining at least one disease parameter and at least one ionizing radiation treatment parameter. In one embodiment, the method of the invention comprises measuring or determining at least one other clinical parameter, at least one disease parameter and at least one ionizing radiation treatment parameter. In one embodiment, the method of the invention further comprise using the age of the patient in the mathematical function.

The method of the invention comprises combining the different values measured or associated with the markers of a) to c)) in a mathematical function to obtain an end-value.

In one embodiment, the method of the invention is non-invasive.

In one embodiment, the subject is a human.

In one embodiment, the subject is treated by ionizing radiation for a prostate cancer. Therefore, the subject is preferably a male. In another embodiment, the subject is not yet treated for prostate cancer when the method if applied.

In one embodiment, said prostate cancer is castrate-resistant prostate cancer, metastatic castrate-resistant prostate cancer, recurrent prostate cancer, unresectable prostate cancer, advanced prostate cancer, or locally advanced prostate cancer.

As used herein, advanced cancer is cancer that has spread to other places in the body and usually cannot be cured or controlled with treatment. In particular, locally advanced cancer is cancer that has spread from where it started to nearby tissue or lymph nodes. Unresectable cancer is a cancer that is unable to be removed with surgery. Recurrent cancer is cancer that has recurred (come back). The cancer may come back to the same place as the original (primary) tumor or to another place in the body. Metastatic cancer is cancer that has spread from the place where it first started to another place in the body. A tumor formed by metastatic cancer cells may be called a metastatic tumor or a metastasis. The metastatic tumor contains cells that are like those in the original (primary) tumor. Castrate-resistant prostate cancer is prostate cancer that keeps growing even when the amount of testosterone in the body is reduced to very low levels. Many early-stage prostate cancers need normal levels of testosterone to grow, but castrate-resistant prostate cancers do not.

The American Joint Committee on Cancer (AJCC) classifies prostate cancer according to a tumor, nodes, metastasis (TNM) classification system for prostate cancer. TNM Stage III includes the criterion that 'Tumor extends through the prostate capsule'. Hence, Stage III prostate cancer is broadly equivalent to locally advanced prostate cancer. TNM Stage IV disease meets the primary tumor criterion that the "tumor is fixed or invades adjacent structures other than seminal vesicles such as external sphincter, rectum, bladder, levator ani, and/or pelvic wall". Hence, stage IV prostate cancer is broadly equivalent to advanced prostate cancer. TNM Stage IV disease is also defined as any occurrence of metastasis in regional lymph node(s) or any occurrence of distant metastasis. Hence, stage IV prostate cancer is broadly equivalent to metastatic prostate cancer.

In one embodiment, prostate cancer is TNM stage I prostate cancer, including Ia, Ib, Ic.

In another embodiment, prostate cancer is TNM stage II prostate cancer.

In another embodiment, prostate cancer is TNM stage III prostate cancer.

In another embodiment, prostate cancer is TNM stage IV prostate cancer.

In one embodiment, said sample is a T cell containing sample.

Examples of T cell containing samples include, but are not limited to, whole blood samples and samples recovered from bone marrow, lymph node tissue, cord blood, thymus tissue, tissue from a site of infection, ascites, pleural effusion, spleen tissue, and tumors. In one embodiment of the present invention, the T cell containing sample can be obtained from a unit of blood collected from a subject using any number of techniques known to the skilled artisan, such as, for example, by leukapheresis.

In one embodiment, said sample is a bodily fluid sample, such as, for example, a blood, plasma, serum, lymph, urine, cerebrospinal fluid or sweat sample.

In one embodiment, said sample is a blood sample.

In one embodiment, the sample is recovered prior to the implementation of the method of the invention, *i.e.*, the step of recovering the sample is not part of the method of the invention.

According to the invention, the subject is, was or will be treated by ionizing radiation.

It is preferred when the subject is planned to be treated by ionizing radiation, and the method of the invention is implemented before the beginning of the TIR.

The term "ionizing radiation", as used herein, refers to a treatment involving the use of high-energy radiation such as, for example, X-rays, gamma rays, electron beams or protons, to kill or damage cancer cells and stop them from growing and multiplying.

In one embodiment, the ionizing radiation involves the use of X-rays.

In one embodiment, the ionizing radiation involves the use of brachytherapy or brachytherapy boost.

The side effects are side effects induced by ionizing radiation in the pelvic area.

Examples of radiation-induced side effects include, but are not limited to, genitourinary (such as, for example, urinary and sexual toxicities), gastrointestinal and neurologic toxicities.

Examples of radiation-induced urinary toxicities include, but are not limited to, dysuria, pollakiuria, abnormal (*e.g.*, increased) urinary frequency, decreased urine stream, burning urination, bleedings, urinary incontinence, mictional or urinary impetuosity, nocturia, hematuria, and urinary pain.

Examples of radiation-induced gastrointestinal toxicities include, but are not limited to, diarrhea, constipation, rectal syndrome, functional proctitis and/or anusitis, physical proctitis and/or anusitis, radiation proctitis, ano-proctitis, bleeding (in particular rectal bleeding or anal bleeding), and gastrointestinal pain (in particular rectal or anal pain).

Examples of radiation-induced sexual toxicities include, but are not limited to, sexual impotence, erectile dysfunction, and ejaculation dysfunction.

Another example of radiation-induced side effects is pelvic neuropathy.

In one embodiment, the method of the invention aims at predicting the risk of developing side effects during ionizing radiation treatment and a period of about 1, 3, 6, 12, 18, 24, 30 or 36 months after TIR. It is particularly adapated to predicting the risk of developing side effects within 24 months after the TIR.

In one embodiment, the *in vitro* method of the invention aims at predicting the risk of developing acute side effects, *i.e.*, side effects occurring during TIR or less than about 1 week, 2 weeks, 3 weeks or 4 weeks after TIR, or less than about 1, 2 or 3 months after TIR. Such acute side effects of the pelvic area may be referred to as Prostate Side Effect (or PSE).

In another embodiment, the *in vitro* method of the invention aims at predicting the risk of developing late side effects, *i.e.*, side effects occurring at least about 3 months after ionizing radiation treatment, such as, for example, between about 3 months and about 6 months after ionizing radiation treatment, between about 3 months and about 12 months after ionizing radiation treatment, between about 3 months and about 18 months after ionizing radiation treatment, between about 3 months and about 2 years after ionizing radiation treatment, between about 3 months and about 30 months after ionizing radiation treatment, or between about 3 months and about 3 years after ionizing radiation treatment. In one embodiment, the late side effects occur about 4, 5, 6, 7, 8, 9, 10, 11, 12 months or more after ionizing radiation treatment, or 2 or 3 years or more after ionizing radiation treatment. In one embodiment, such late side effects of the pelvic area may be referred to as Prostate Side Effect (or PSE).

In one embodiment, the side-effects as listed hereinabove are at least Grade 2 side effects according to the CTCAE, in particular to the v3.0 CTCAE. In one embodiment, the side-effects as listed hereinabove are Grade 2, Grade 3, Grade 4 or Grade 5 side effects according to the CTCAE, in particular to the v3.0 CTCAE, preferably Grade 2, Grade 3, or Grade 4.

In one embodiment, said at least one other biochemical marker measured in step (a) is selected from the group comprising proteins of radiosensitivity and genes of radiosensitivity.

The method of the invention comprises measuring RILA (wherein RILA stands for "radiation-induced T-lymphocytes apoptosis") in a sample of the subject. In one embodiment, the method of the invention comprises measuring radiation-induced apoptosis of CD4 and/or CD8 T-lymphocyte. In one embodiment, the method of the invention comprises measuring radiation-induced CD8 T-lymphocyte apoptosis.

RILA is well known from the man skilled in the art and was described in Ozsahin et al., 1997 and 2005. RILA corresponds to the difference of percentage (%) between the rate of radiation-induced T lymphocytes apoptosis after an irradiation of a T lymphocyte containing sample of the subject (preferably a blood sample), with X-Rays (*e.g.*, 8 Gy X-Rays) and the rate of lymphocytes apoptosis without any irradiation (0 Gy). In one embodiment, T lymphocytes are CD8 lymphocytes. In another embodiment, T lymphocytes are CD4 lymphocytes. In summary, the RILA test consists in irradiating lymphocytes of the patient and detecting the apoptosis rate. In particular, one applies a dose equivalent to 8Gy to lymphocytes (preferably a 1Gy/mn) on lymphocytes of a blood sample (between 24 and 48 hours after harvest) and apoptosis rate is determined after sample incubation after such application (48 hours when irradiated with 8Gy). The lymphocytes for which apoptosis rate is evaluated are preferably CD8 lymphocytes. Apoptosis rate is evaluated by methods known in the art, and in particular using flow cytometry.

Therefore, in one embodiment, the method of the invention comprises, in step (a), measuring the difference of percentage (%) between the rate of radiation-induced T lymphocytes (preferably CD8 T lymphocytes) apoptosis after an irradiation of a T lymphocyte containing sample of the subject (preferably a blood sample), with 8 Gy X-Rays and the rate of lymphocytes apoptosis without any irradiation (0 Gy).

In one embodiment, the rate of T lymphocytes apoptosis with and without any radiation is measured according to RILA assay which is commonly known by the man skilled in the art, and which is described in Ozsahin et al., 1997 or 2005.

In one embodiment, such RILA assay may thus comprise the following steps:

i. cell culture of the cells comprised in the T cell containing sample (preferably blood sample), wherein said T cell containing sample (preferably blood sample) is preferably recovered from the subject prior to the implementation of the method of the invention;

ii. sample irradiation at 8Gy, such as, for example, using a linear accelerator or an experimental irradiator; and

iii. sample labelling for evaluation of T lymphocytes (preferably CD8 T-lymphocytes) apoptosis.

In one embodiment, the evaluation of T-lymphocytes (preferably CD8 T-lymphocytes) apoptosis at the step iii) may use a method selected from the group comprising, but not limited to, FACS analysis, dosage of Annexin 5, and dosage of caspases, preferably the evaluation of T lymphocytes apoptosis is carried out by FACS analysis.

In one embodiment, at least one T cell containing sample (preferably blood sample) is collected from the subject before ionizing radiation, preferably in a heparinized tube.

In one embodiment, for cell culture of cells contained in the T cell containing sample (preferably blood sample), a volume of said sample is aliquoted into well plate, such as, for example, into a 6 well tissue culture plate. Culture medium may thus be added onto the culture plate, such as, for example, 2 ml of medium RPMI1640 (Gibco), supplemented with 20% FCS (fetal calf serum). Cells may thus be cultured for 15 to 30 hours, preferably for 24 hours, more preferably at 37°C, 5% $CO_2$ before irradiation.

For irradiation studies, the assay may be carried out in triplicate.

In one embodiment, after culture, cells of the T cell containing sample may be irradiated using a linear accelerator or an experimental irradiator.

In one embodiment, after culture, cells of the T cell containing sample may be irradiated at 8 Gy using a linear accelerator, with a dose rate ranging from 0.1 to 10 Gy/minute, preferably of about 1 Gy/minute.

In one embodiment, the conditions of irradiation in a linear irradiator are:

- Dose rate = 1Gy/min
- Energy = 6MV
- Depth = 15 mm polystyrene on cells
- DSP (Distance between Source and Plate) = 145 cm
- Field = 25 * 25 $cm^2$ collimator
- Linear accelerator (machine output: 200 UM/min): 200 UM for 1Gy: 1600 UM for 8Gy.

In one embodiment, control cells (*i.e.*, cells treated with 0 Gy) are removed from the incubator and kept for the same period of time under the same conditions, but without TIR.

In one embodiment, after irradiation (or after control treatment), cells are incubated for 40 to 50 hours, preferably for about 48 hours, at 37°C, 5% $CO_2$.

In one embodiment, after 40 to 50 hours of incubation, preferably after about 48 hours of incubation, culture plates are placed at room temperature (*e.g.*, about 15 to about 30°C, preferably about 18 to about 25°C), and the content of each well is transferred to pre-labeled centrifuge tubes, for centrifugation, such as, for example, for 5 minutes at 1450 rpm. After centrifugation, the sample may be labeled with anti-human CD4 and/or anti-CD8-FITC antibody, preferably with CD8-FITC antibody. Then, lysis buffer is added to the centrifuge tube in order to lyse any non-lymphocyte cell (such as, for example, red blood cells). An example of lysis buffer is the ammonium chloride based lysing reagent provided by Becton Dickinson (USA). After lysis, reagents are added to the tube for evaluating lymphocytes apoptosis according to usual methods known from the man skilled in the art. Examples of such methods include, but are not limited to, flow cytometry (FACS) with propidium iodide and RNase A as reagents.

The method of the invention comprises determining the value of the RILA test, and combining said value with a value associated with the presence or absence of urinary toxicity in the patient, and optionally with values associated with at least one other biochemical parameter, one other clinical parameter, one disease parameter and/or one ionizing treatment parameter.

In one embodiment, at least one other biochemical marker is measured and used in the function. Said at least one other biochemical marker can be a protein of radiosensitivity, preferably selected from the group consisting of AK2 (adenylate kinase 2), HSPA8 (Heat shock cognate protein 71 kDa, also referred to as HSC70), ANX1 (Annexin 1), APEX1 (DNA-(apurinic or apyrimidinic site) lyase) and ID2 (mitochondrial isocitrate dehydrogenase 2, that may also be referred to as IDH2), fragments and combinations thereof.

In one embodiment, said at least one other biochemical marker is a combination of at least two proteins of radiosensitivity, of at least three proteins of radiosensitivity, of at least four proteins of radiosensitivity, or of five proteins of radiosensitivity, preferably selected from the group consisting of AK2, HSPA8, ANX1, APEX1 and ID2.

As used herein, a "protein of radiosensitivity" refers to a protein whose expression (either at the protein or RNA level) is indicative of the radiosensitivity of the subject.

Consequently, in one embodiment, measuring at least one other biochemical marker at step (a) corresponds to measuring a protein level of at least one protein of radiosensitivity in a sample from the subject or to measuring a nucleic acid encoding said protein in a sample from the subject. In one embodiment, in a first step, proteins and/or nucleic acids are isolated from a biological sample previously obtained from the subject. A method according to the invention may thus include protein or nucleic acid extraction, purification and characterization, using well known biochemistry methods.

The presence or level of said protein of radiosensitivity may be determined by methods well known in the art. Examples of such methods include, but are not limited to, a method based on immune-detection, a method based on western blot, a method based on mass spectrometry, a method based on chromatography, or a method based on flow cytometry, and a method for specific nucleic acid detection. Specific examples of *in vitro* methods for determining a protein level in a sample are well-known in the art, and include, but are not limited to, immunohistochemistry, Multiplex methods (Luminex), western blot, enzyme-linked immunosorbent assay (ELISA), sandwich ELISA, fluorescent-linked immunosorbent assay (FLISA), enzyme immunoassay (EIA), radioimmunoassay (RIA), flow cytometry (FACS) and the like.

In one embodiment, the presence and level of expression of proteins can be determined directly or be analyzed at the nucleic level by detecting, and preferably quantifying, protein-specific nucleic acids, and particularly mRNA (*i.e.*, assessing the transcription level of the protein). Methods for assessing the transcription level of a protein are well known in the prior art. Examples of such methods include, but are not limited to, RT-PCR, RT-qPCR, Northern Blot, hybridization techniques such as, for example, use of microarrays, and combination thereof including but not limited to, hybridization of amplicons obtained by RT-PCR, sequencing such as, for example, next-generation DNA sequencing (NGS) or RNA-seq (also known as "Whole Transcriptome Shotgun Sequencing") and the like.

In one embodiment, said at least one biochemical marker is a gene of radiosensitivity, preferably selected from the group consisting of TGFβ, SOD2, TNFα, and XRCC1.

As used herein, a "gene of radiosensitivity" refers to a gene whose expression (either at the protein or RNA level) is indicative of the radiosensitivity of the subject, or to a gene comprising at least one single nucleotide polymorphism (SNP) indicative of the radiosensitivity of the subject, or involved in the fibrosis pathway and ROS management.

Consequently, in one embodiment, measuring at least one other biochemical marker at step (a) corresponds to measuring an expression level of or determining the presence of a SNP in at least one gene of radiosensitivity in a sample from the subject. In this embodiment, the value associated with the presence (or absence) of the SNP will be 1 (or 0). Alternatively, if the expression level is measured, the measured value can be used in the formula.

The presence or level of expression of said gene of radiosensitivity may be determined by a usual method known from the person skilled in the art. A nonlimiting list of such methods is shown hereinabove.

In one embodiment, the method for determining presence or level of expression of a gene of radiosensitivity is as disclosed in Azria et al., 2008 and includes lymphocyte isolation, DNA extraction and amplification, and denaturing high-performance liquid chromatography or the Surveyor nuclease assay using a Transgenomic WAVE High Sensitivity Nuclei Acid Fragment Analysis System. PCR primers for the DNA amplicons encompassing the SNPs of interest disclosed above may designed using, for example, the genomic sequence obtained from the NCBI.

In one embodiment, the method of the invention comprises measuring at step (a) RILA and at least one protein of radiosensitivity. In another embodiment, the method of the invention comprises measuring at step (a) RILA and at least one gene of radiosensitivity. In another embodiment, the method of the invention comprises measuring at step (a) RILA, at least one protein of radiosensitivity and at least one gene of radiosensitivity.

It is also interesting to use, as one other biochemical marker, the level of Prostate Specific Antigen (PSA) in the blood of the patient.

By "clinical parameter" it is meant any clinical parameter related to the subject and relevant to assess an increased risk of radiation-induced toxicity in said subject. One clinical parameter (presence of urinary toxicity) is determined according to the present disclosure.

Examples of clinical parameters include, but are not limited to, concomitant medical treatment (*e.g.*, neoadjuvant, concomitant and/or adjuvant hormonotherapy or anticoagulant therapy), age, presence or history of other diseases or conditions, preferably before ionizing radiation (which may also be referred to as the presence of at least one comorbidity, wherein said comorbidities may be selected, without limitation, from high blood pressure, diabetes, angina pectoris, heart failure, arteritis, chronic hepatitis, respiratory failure, sexual disorders, digestive disorders), PSA (prostate-specific antigen) level, and tobacco smoking.

In particular, it is determined that the patient has urinary toxicity (or urinary disorder) if the patient has at least one of dysuria (painful urination), urine color change (in particular hematuria), pollakiuria, bladder spasms, urinary frequency/urgency, urinary retention, urinary leak, incontinence, obstruction or stricture/stenosis (bladder, ureter or urethra), urinary tract infection (such as cystitis) and/or erectile dysfunction and when the trouble is graded 1 or more, according to the CTCAE v3.0 when relevant. Other signs of urinary toxicity may be used.

It is also possible to use, as the marker of urinary toxicity, only one of the above marker, in particular dysuria.

If the patient presents no urinary toxicity, the value associated with this presence of the clinical marker in the function is 0.

If the patient presents such urinary toxicity, the value associated with this presence of the clinical marker in the function depends on the value of the grade as determined in the CTCAE v3.0.

Alternatively, one can assess the value 1 in case of presence of urinary toxicity, whatever the grade of such.

In one embodiment, at least one other clinical marker is used in the function. In particular, such marker is presence (or absence) of a gastro-intestinal disorder.

In particular, it is determined that the patient has gastro-intestinal toxicity (or gastro-intestinal disorder) if the patient has at least one of diarrhea, hemorrhoids, rectum inflammation (whether functional or physical) and when the trouble is graded 1 or more, according to the CTCAE v3.0 when relevant.

It is also possible to use, as the marker of gastro-intestinal toxicity, only one of the above marker, in particular rectum inflammation.

If the patient presents such gastro-intestinal toxicity, the value associated with this presence of the clinical marker in the function depends on the value of the grade as determined in the CTCAE v3.0.

If the patient presents no gastro-intestinal toxicity, the value associated with this presence of the clinical marker in the function is 0.

In one embodiment, another clinical marker is used in the function. In particular, such at least one other clinical marker is presence (or absence) of diabetes in the patient.

In one embodiment, another clinical marker is used in the function. In particular, such at least one other clinical marker is presence (or absence) of a gastro-intestinal disorder, preferably also taking into consideration the grade of such.

In one embodiment, another clinical marker is used in the function. In particular, such at least one other clinical marker is presence (or absence) of intake of anticoagulant by the patient, in particular linked to high blood pressure.

In one embodiment, the at least one other clinical parameter measured in step (b) is neoadjuvant, concomitant and/or adjuvant hormonotherapy. As used herein, the term "neoadjuvant, concomitant and/or adjuvant hormonotherapy" refers to a treatment given after surgery, chemotherapy, and/or radiation therapy to lower the risk of recurrence of the cancer.

Examples of neoadjuvant, concomitant and/or adjuvant hormonotherapy include, but are not limited to, anti-androgens administration, administration of androgen synthesis inhibitors or administration of agonists of LHRH (luteinizing hormone-releasing hormone) or administration of GnRH antagonist. Examples of anti-androgens include, but are not limited to, flutamide, bicalutamide, nilutamide and enzalutamide. Examples of androgen synthesis inhibitors include, but are not limited to, ketoconazole, aminoglutethamide, abiraterone acetate and enzalutamide. Examples of LHRH agonists include, but are not limited to, leuprolide, goserelin, triptorelin and histrelin. Examples of GnRH antagonists include, but are not limited to, abarelix, degarelix, ozarelix and relugolix.

In one embodiment, the at least one clinical parameter measured in step (b) is the presence of at least one comorbidity before treatment, preferably selected from the group comprising high blood pressure, diabetes, angina pectoris, heart failure, chronic hepatitis, respiratory failure, arteritis, sexual disorders, and digestive disorders (disorders of the gastro-intestinal system).

In one embodiment, the at least one clinical parameter measured in step (b) is selected from the group comprising neoadjuvant, concomitant and/or adjuvant hormonotherapy, tobacco smoking and the presence of at least one comorbidity.

In one embodiment, for clinical parameter "tobacco smoking", a tobacco smoking patient is defined consistently as daily smoker, intermittent smoker or non-daily smoker (and given value=1 in the mathematical function, preferably in the multivariate Cox function), while a non-smoking patient is defined as some-day smoker or never daily smoker (and given value=0 in the mathematical function, preferably in the multivariate Cox function).

By "disease parameter" it is meant any parameter related to the disease (*e.g.*, prostate cancer) of the subject and relevant to assess an increased risk of radiation-induced toxicity in said subject. Examples of disease parameters include, but are not limited to, Gleason score, T grade (tumors according to the TNM classification), N grade (nodes according to the TNM classification), clinical characteristics of the prostate tumor (*e.g.*, size or volume of the tumor), and presence or absence of metastasis.

The Gleason grading system is based on microscopic appearance of prostate cancer. Gleason scores range from 2 to 10, with 2 representing the most-differentiated tumors and 10 the least-differentiated tumors. Tumors with Gleason scores of 8 to 10 are generally advanced neoplasms with low chances of cure.

Using the TNM system, the "T" plus a letter or number (0 to 4) is used to describe the size and location of the tumor. Some stages are also divided into smaller groups that help describe the tumor in even more detail.

The different T grades are the following: T0: There is no evidence of a tumor in the prostate; T1: The tumor cannot be felt during a digital rectal exam and is not seen during imaging tests. It may be found when surgery is done for another reason, usually for benign prostatic hyperplasia or an abnormal growth of noncancerous prostate cells. T1a: The tumor is in 5% or less of the prostate tissue removed during surgery. T1b: The tumor is in more than 5% of the prostate tissue removed during surgery. T1c: The tumor is found during a needle biopsy, usually because the patient has an elevated PSA level. T2: The tumor is found only in the prostate, not other parts of the body. It is large enough to be felt during a digital rectal exam. T2a: The tumor involves one-half of 1 lobe (part or side) of the prostate. T2b: The tumor involves more than one-half of 1 lobe of the prostate but not both lobes. T2c: The tumor has grown into both lobes of the prostate. T3: The tumor has grown through the prostate capsule on 1 side and into the tissue just outside the prostate. T3a: The tumor has grown through the prostate capsule either on 1 side or on both sides of the prostate, or it has spread to the neck of the bladder. This is also known as an extraprostatic extension (EPE). T3b: The tumor has grown into the seminal vesicle(s), the tube(s) that carry semen. T4: The tumor is fixed, or it is growing into nearby structures other than the seminal vesicles, such as the external sphincter, the part of the muscle layer that helps to control urination; the rectum; levator ani; or the pelvic wall.

In the TNM system, the "N" stands for lymph nodes. These bean-shaped organs help fight infection. Lymph nodes near the prostate in the pelvic region are called regional lymph nodes. Lymph nodes in other parts of the body are called distant lymph nodes. The different N grades are the following: N0: The cancer has not spread to the regional lymph nodes. N1: The cancer has spread to the regional (pelvic) lymph node(s).

In one embodiment, for clinical parameter "Gleason", a Gleason $\leq 7$ is given value=0 in the mathematical function, preferably in the multivariate Cox function, while a Gleason > 7 is given value=1 in the mathematical function, preferably in the multivariate Cox function.

In one embodiment, for clinical parameter "T grade", a T grade of 0 is given value=0 in the mathematical function, preferably in the multivariate Cox function, while a T grade of 1 to 4 is given value=1 in the mathematical function, preferably in the multivariate Cox function. Alternatively, specific values can be given for each grade above 0.

In one embodiment, for clinical parameter "N grade", a N grade of 0 s given value=0 in the mathematical function, preferably in the multivariate Cox function, while a N grade of 1 or 2 is given value=1 in the mathematical function, preferably in the multivariate Cox function. Alternatively, specific values can be given for each grade above 0.

By "ionizing radiation treatment parameter" it is meant any parameter related to the ionizing radiation treatment planned to be applied to the subject and relevant to assess an increased risk of radiation-induced toxicity in the subject. Examples of ionizing radiation treatment parameters include, but are not limited to, ionizing radiation treatment energy range (*e.g.*, ranging from 6 to 25 MV), number of beam(s), technic used (either Intensity-modulated radiation therapy (IMRT) or 3D conformational radiation therapy (3D-RT)), boost (complement dose of irradiation), node irradiation, CTV, CTV1, CTV2, PTV, PTV1, PTV2, PTV1 maximal dose, PTV1 minimal dose, PTV1 mean dose, volume receiving 44 Gy, PTV2 maximal dose, PTV2 minimal dose, PTV2 mean dose, minimal dose received by 85% of the prescribed dose, volume PTV2 receiving 95% of the prescribed dose, volume of the rectal wall, volume of the bladder wall, volume of the intestinal contour, intestinal volume, maximal dose received by the rectal wall, dose received by 25% of the rectal volume, maximal dose received by the bladder wall, dose received by 25 or 50% of the bladder volume, dose received by 5% of the right or left femoral head, dose received by 70% of the penis bulb, mean dose received at the strip, treated volume (50 or 95% of the dose), volume of the anal canal, anal canal maximal dose, dose received by 25% or 50% of the volume of the anal canal.

In one embodiment, the method of the invention comprises measuring CTV, CTV1 and/or CTV2 in the subject.

The terms PTV and CTV are well known from the clinicians. The clinical target volume (CTV) comprises the position and extent of gross tumor, *i.e.*, what can be seen, palpated or imaged (which can be referred to as the gross tumor volume), plus a margin for sub-clinical disease spread which therefore cannot be fully imaged.

The planning target volume (PTV), allows for uncertainties in planning or treatment delivery. It is a geometric concept designed to ensure that the ionizing radiation dose is actually delivered to the CTV.

In one embodiment, the planning target volume (PTV) 1 may be defined as the prostate and seminal vesicles (proximal part in the absence of invasion) plus a 1 cm margin in all directions except posteriorly (5 mm), plus the CTV for lymph nodes and a 7 mm set-up margin when indicated. In case of a post-prostatectomy ionizing radiation, the planning target volume (PTV) 1 may be defined as the prostate bed plus a 1 cm margin in all directions except posteriorly (5 mm), plus the CTV for lymph nodes and a 7 mm set-up margin when indicated.

In one embodiment, the PTV2 consists of the prostate gland only or the prostate gland plus invaded seminal vesicles, with a 1 cm (5 mm posteriorly) margin. In case of a post-prostatectomy ionizing radiation, the PTV2 may consist of the prostate bed only with a 1 cm (5 mm posteriorly) margin.

In one embodiment, ionizing radiation treatment parameters as listed hereinabove are continued data, in particular parameters related to volumes, doses, etc...

In one embodiment, for the clinical parameters, disease parameters or ionizing radiation treatment parameters as listed hereinabove and that may be present or absent, the presence of said parameter is given value=1 in the mathematical function, preferably in the multivariate Cox function, while its absence is given value=0 in the mathematical function, preferably in the multivariate Cox function. For example, in one embodiment, at step (b), if the subject is treated by neoadjuvant, concomitant and/or adjuvant hormonotherapy, the value "1" is affected. In one embodiment, at step (b), if the subject is not treated by neoadjuvant, concomitant and/or adjuvant hormonotherapy, the value "0" is affected to the subject. In another embodiment, for continued data, the parameter is given its exact value in the mathematical function, preferably in the multivariate Cox function.

It is preferred to use a function that combines at least RILA, presence/absence of a urinary disorder and tumor T grade.

It is preferred to use a function that combines only RILA, presence/absence of a urinary disorder and tumor T grade.

It is preferred to use a function that combines at least RILA, presence/absence of a urinary disorder, tumor T grade and presence/absence of a gastro-intestinal disorder.

It is preferred to use a function that combines at least RILA, presence/absence of a urinary disorder, tumor T grade and presence/absence of diabetes.

It is preferred to use a function that combines at least RILA, presence/absence of a urinary disorder, tumor T grade, presence/absence of diabetes, and presence/absence of a gastro-intestinal disorder.

It is preferred to use a function that combines at least RILA, presence/absence of a urinary disorder, tumor T grade, presence/absence of diabetes, and presence/absence of a gastro-intestinal disorder.

It is preferred to use a function that combines at least RILA, presence/absence of a urinary disorder, tumor T grade, age of the patient, presence/absence of diabetes, and presence/absence of a gastro-intestinal disorder.

It is preferred to use a function that combines at least RILA, presence/absence of a urinary disorder, tumor T grade, age of the patient, and presence/absence of a gastro-intestinal disorder.

It is preferred to use a function that combines at least RILA, presence/absence of a urinary disorder, tumor T grade, level of PSA, and presence/absence of a gastro-intestinal disorder.

It is preferred to use a function that combines at least RILA, presence/absence of a urinary disorder, tumor T grade, tumor N gradde, and presence/absence of a gastro-intestinal disorder.

It is preferred to use a function that combines at least RILA, presence/absence of a urinary disorder, tumor T grade, intake of anticoagulants, presence/absence of diabetes, and presence/absence of a gastro-intestinal disorder.

It is preferred to use a function that combines at least RILA, presence/absence of a urinary disorder, tumor T grade, level of PSA, presence/absence of diabetes, and presence/absence of a gastro-intestinal disorder.

It is preferred to use a function that combines at least RILA, presence/absence of a urinary disorder, tumor T grade, intake of anticoagulants, and presence/absence of diabetes.

It is preferred to use a function that combines at least RILA, presence/absence of a urinary disorder, tumor T grade, age of the patient, and presence/absence of a gastro-intestinal disorder.

It is preferred to use a function that combines at least RILA, presence/absence of a urinary disorder, tumor T grade, and level of PSA.

It is preferred to use a function that combines at least RILA, presence/absence of a urinary disorder, and presence/absence of a gastro-intestinal disorder.

It is preferred to use a function that combines at least RILA, presence/absence of a urinary disorder, and presence/absence of diabetes.

It is preferred to use a function that combines at least RILA, presence/absence of a urinary disorder, and age of the patient.

The present description also provides methods for obtaining a mathematical function that can be used in an in vitro non-invasive diagnosis test for assessing the risk of developing side effects after ionizing radiation in a subject suffering from prostate cancer.

These methods are performed on a cohort of patients that have received ionizing radiation treatment and for which occurrence of PSE have been reorded post-treatment.

These methods include:

a) classifying subjects of a cohort of patients into different groups according to the presence of side effects after ionizing radiation

b) providing the value at least one biochemical marker in a sample of each subjects, wherein said at least one biochemical marker is radiation induced T-lymphocytes apoptosis (RILA) measured before ionizing radiation treatment; and

c) determining the presence or absence of at least one clinical parameter, disease parameter from each patient, wherein said at least one clinical parameter, disease parameter comprises presence of urinary toxicity prior to ionizing radiation treatment

d) optionally determining the presence of at least one other marker selected from the group consisting in the presence or absence of concomitant and/or adjuvant hormonotherapy, the tobacco smoking habit, the presence of gastrointestinal toxicity, the dosage of PSA (Prostate Specific Antigen), the tumor T stage, the tumor N stage, the presence of at least one comorbidity (in particular diabetes) and the age of the patient, before ionizing radiation treatment

e) identifying by unidimensional analysis, the markers measured in step d) for which the measured value differs significantly between the group of subjects in which side effects after ionizing radiation have developed and the group of subjects in which side effects after ionizing radiation have not occurred, thereby obtaining independent factors

f) obtaining the mathematical function by combination of the factor measured in b), the marker determined in c) and the identified independent factors identified in e).

In order to obtain a function that is as accurate as possible, the number of patients in the cohort should be as large as possible, indicating that it preferably comprises more than 50 patients, preferably more than 100 patients, preferably more than 200 patients, more preferably more than 500 patients, or even more than 1000 patients. As indicated, there is no upper limit for the number of patients, and the larger, the better.

The duration of time is chosen by the person skilled in the art. If one desires that the function is indicative of the occurrence of side effect post treatment at two years, the duration of time shall be at least 2 years. This means that the groups of e) will be the patients with PSE after 2 years and the patients without PSE after 2 years. Other time periods can be used (1, 3, 5 years or more). In the present application, the disclosed functions have been designed with a 2 years period. Other functions can be designed, with other endpoint durations, which may be more accurate for these specific endpoints.

As indicated above the function may be a (multivariate) Cox function. Such Cox function is preferably obtained by

a) Assessing / evaluating the presence of PSE after a given duration of time in patients having had prostate cancer and having been treated by ionizing radiation treatment, wherein the values of the variables used in the function are known at the beginning of the duration of time (prior to treatment)

b) Performing a multivariate Cox regression by combination of said values, on the basis of the occurrence of PSE after the given duration of time

c) Assessing the independent discriminative value of the values by analysis of the multivariate Cox regression,

d) Combining the relative weight of the values of each marker, as individually determined in the multivariate Cox regression, with a negative sign when the markers harbor a negative correlation with the observation of PSE.

The markers that can be used in this method are the same as disclosed above. The period of time is chosen by the one of skill in the art, and is generally multiple years (such as 1, 2, 3 or 5 years).

It is to be noted that the different parameters that are proposed are either continuous (RILA, level of circulating PSA, age of the patient), or discrete with a binary classification (presence/absence of a comorbidity such as diabetes) or a classification in multiple classes (such as the classification of the tumor grade according to the TNM classification).

Consequently, the function obtained takes into consideration these differences between the inputed parameters. This implies that the values, in the function, for the parameters classified in discrete classes will depend on the class of the parameter.

As an illustration, for a function involving RILA (parameter a) a presence of urinary toxicity (parameter b), a single coefficient a1 will be used and multiplied by the value found for RILA (thereby representing a proportional contribution of RILA in the function), and various coefficients b1, b2, b3, b4 will be used depending on the stage of the urinary toxicity according to the CTCAE (grades 1 to 4 respectively).

In one embodiment, the *in vitro* method for assessing the risk of developing side effects after ionizing radiation in a subject, thus comprises the steps of:

a) measuring RILA in a sample of the subject;

b) determining the presence and optionally the grade of urinary disorders for the subject
c) optionally measuring or determining at least one other clinical parameter, disease or ionizing radiation treatment parameter for the subject.
d) combining values associated with the parameters measured or determined in a), b) and optionally c) in a mathematical function in order to obtain an end value.

In one embodiment, the *in vitro* method for assessing the risk of developing side effects after ionizing radiation in a subject, comprises the steps of:

a) measuring RILA in a sample of the subject;
b) determining the presence and optionally the grade of urinary disorders for the subject
c) determining the tumor T grade according to the TNM classification
d) combining values associated with the parameters measured or determined in a), b) and c) in a mathematical function in order to obtain an end value.

In a specific embodiment, the age of the subject is also included in the mathematical function of d).
In one embodiment, the *in vitro* method for assessing the risk of developing side effects after ionizing radiation in a subject, comprises the steps of:

a) measuring RILA in a sample of the subject; and
b) determining the presence and optionally the grade of urinary disorders for the subject
c) determining the tumor T grade according to the TNM classification
d) determining the presence and optionally the grade of gastro-intestinal disorders for the subject
e) combining values associated with the parameters measured or determined in a), b), c) and d) in a mathematical function in order to obtain an end value.

In a specific embodiment, the age of the subject is also included in the mathematical function of e).
In one embodiment, the in vitro method for assessing the risk of developing side effects after ionizing radiation in a subject, comprises the steps of:

a) measuring RILA in a sample of the subject; and
b) determining the presence and optionally the grade of urinary disorders for the subject
c) determining the tumor T grade according to the TNM classification
d) determining the presence or absence of a comorbidity, preferentially diabetes and/or intake of anticoagulants
e) combining values associated with the parameters measured or determined in a), b), c) and d) in a mathematical function in order to obtain an end value.

In a specific embodiment, the age of the subject is also included in the mathematical function of e).
In one embodiment, the in vitro method for assessing the risk of developing side effects after ionizing radiation in a subject, comprises the steps of:

a) measuring RILA in a sample of the subject; and
b) determining the presence and optionally the grade of urinary disorders for the subject
c) determining the tumor T grade according to the TNM classification
d) determining the presence or absence of a comorbidity, preferentially diabetes and/or intake of anticoagulants
e) determining the presence and optionally the grade of gastro-intestinal disorders for the subject
f) combining values associated with the parameters measured or determined in a), b), c), d) and e) in a mathematical function in order to obtain an end value.

In a specific embodiment, the age of the subject is also included in the mathematical function of e).
In one embodiment, the mathematical function of the invention is a binary logistic regression, a multiple linear regression or any time-dependent regression.
In a preferred embodiment, the regression is a multivariate Cox regression.
In one embodiment, said regression is time-dependent, preferably is a time-dependent multivariate regression.
In one embodiment, said regression is a multivariate time-related model, preferably a Cox proportional hazard regression model. Such model is particularly well adapted for the purpose of the present invention, namely making a prognosis of the occurrence of an event (assessing the risk (or hazard) of the occurrence of such event) over time or after a given period of time, said event being the occurrence of side effect linked to the radiation treatment.

In one embodiment of the present invention, the independent parameters combined in the Cox regression are RILA, presence (optionally and preferably grades) of urinary disorders, and preferably tumor T grade (according to TNM classification). Use of biochemical markers, clinical parameters, disease parameters and/or ionizing radiation treatment parameters related to the development of radiation-induced side effects, *e.g.*, PLE in a subject, *e.g.*, RILA, neoadjuvant, concomitant and/or adjuvant hormonotherapy (0 if absent, 1 if present), tobacco smoking, comorbidities, and/or ionizing radiation treatment parameters (in particular CTV, CTV1 and/or CTV2), as indicated above is also possible and the functions (and coefficient thereof) would be adjusted and modified depending on the other parameters included.

In one embodiment, the independent parameters combined in the Cox regression are RILA, presence (or grade) of urinary disorders, and optionally other biochemical markers, clinical parameters, disease parameters and/or ionizing radiation treatment parameters related to the development of radiation-induced side effects, as indicated above.

The multivariate Cox function may usually be obtained by combining the relative weight of each parameter, as individually determined in the multivariate Cox regression, with a negative sign when the markers harbor a negative correlation with the observation of PSE.

As indicated above, in order to define the multivariate Cox model of the invention (*i.e.*, "modelling"), a classification of cancer patients (preferably prostate cancer patients) is made based on the detection of radiation-induced side effects, preferably of PSE as described hereinabove, during the clinical follow-up of studies.

In one embodiment, said modelling may be based on a population (*e.g.*, a multicenter population) of prostate cancer patients treated by ionizing radiation (which may be named "reference population"). The steps to build up the model may thus consist in:

- the identification of biochemical markers and of clinical parameters, disease parameters or ionizing radiation treatment parameters relevant to assess an increased risk of radiation toxicity in a subject;
- the use of said identified biochemical markers and clinical parameters, disease parameters or ionizing radiation treatment parameters in a multicenter clinical trial to identify relevant variables as prognostic factors of radiation-induced side effects, in particular PSE, *i.e.*, as variables that are indicative of a specific risk to develop radiation-induced side effects, in particular PSE;
- the application of these variables on the large multicenter clinical trial to identify the predictive role of the combination of biochemical markers (*e.g*, RILA) and of clinical parameters, disease parameters or ionizing radiation treatment parameters for developing radiation-induced side effects, in particular PSE.

By "multicenter research trial" it is meant a clinical trial conducted at more than one medical center or clinic.

In one embodiment, the multivariate Cox model is:

Hazard (not experiencing a radiation-induced side effect at time t) = baseline hazard at time t*exp(($\alpha$1*RILA) + $\beta$1*(Clinical parameter or disease parameter or ionizing radiation treatment parameter 1) + $\beta$2*(Clinical parameter or disease parameter or ionizing radiation treatment parameter 2) + ... $\beta$n*(Clinical parameter or disease parameter or ionizing radiation treatment parameter (n) with n superior or equal to 2), where the baseline hazard corresponds to the hazard of not experiencing the event (the event being having PSE, not experiencing the event is remaining survival PSE-free) when all covariates are zero.

In another embodiment, the multivariate Cox model is:

Probability (experiencing a radiation-induced side effect before time t) = 1 - Probability (not experiencing a radiation-induced side effect before time t)

$$= 1 - (\exp(-\text{Cumulative baseline Hazard at time t}))^{\exp((\alpha 1^* \text{ RILA}) + \beta 1^*(\text{Clinical parameter or disease parameter or ionizing radiation treatment parameter 1}) + \beta 2^*(\text{Clinical parameter or disease parameter or ionizing radiation treatment parameter 2}) + \dots \beta n^*(\text{Clinical parameter or disease parameter or ionizing radiation treatment parameter (n) with n superior or equal to 2})}$$

$$= 1 - a^{\exp((\alpha 1^* \text{ RILA}) + \beta 1^*(\text{Clinical parameter or disease parameter or ionizing radiation treatment parameter 1}) + \beta 2^*(\text{Clinical parameter or disease parameter or ionizing radiation treatment parameter 2}) + \dots \beta n^*(\text{Clinical parameter or disease parameter or ionizing radiation treatment parameter (n) with n superior or equal to 2})}$$

As indicated above, in the context of the formulas of the present application, a given parameter can be classified in discrete classes X1 to Xm. In this case, the formula will include m coefficients β for this class (for instance β1 to βm). The value associated with each βi coefficient will be "if said parameter is in class i, then value of "Clinical parameter or disease parameter or ionizing radiation treatment parameter" is equal to 1, whereas it is equal to 0 if said parameter isn't in class i.

As an illustration, when there are 4 classes for a parameter, if the parameter for the patient is in class 3, only the β3 coefficient will be taken into consideration, the other ones β1, β2 and β4 being nullified. This is in particular valid for the urinary toxicity included in the formula, as the symptoms can be classified in four classes according to the CTCAE.

The right-hand side of the above equation specify the underlying function of the model. The left-hand side of the equation is the predicted probability. It may be presented in a nomogram and communicated to the patient, in particular to provide an informed consent to the proposed and selected therapy. Alpha and beta coefficients must be estimated for each covariate and converted to hazard ratios as a measure of effect, as in any statistical report. To obtain the predicted probability of the event in question (experiencing a radiation-induced side effect,), the above equation is calculated using a patient's individual characteristics and the model-derived alpha and beta coefficients.

In one embodiment, the baseline hazard at time t corresponds to the basal risk to develop a radiation-induced side effect, with all co-variables considered as reference value (equal to 0).

Clinical parameters '1' to 'n' may be selected in the list of clinical parameters or disease parameters or ionizing radiation treatment parameters as disclosed hereinabove. In one embodiment, said clinical parameters or disease parameters or ionizing radiation treatment parameters include neoadjuvant, concomitant and/or adjuvant hormonotherapy, tobacco smoking, at least one comorbidity, and/or at least one ionizing radiation treatment parameter (in particular CTV, CTV1 and/or CTV2). In one embodiment, said clinical parameters or disease parameters or ionizing radiation treatment parameters include neoadjuvant, concomitant and/or adjuvant hormonotherapy, at least one comorbidity, and/or at least one ionizing radiation treatment parameter (in particular CTV, CTV1 and/or CTV2). In one embodiment, said clinical parameters or disease parameters or ionizing radiation treatment parameters include neoadjuvant, concomitant and/or adjuvant hormonotherapy.

In one embodiment, Hazard (experiencing a radiation-induced side effect) = 1-a^exp((α1* biochemical marker) + β1*(Clinical parameter or disease parameter or ionizing radiation treatment parameter 1) + β2*(Clinical parameter or disease parameter or TIR parameter 2) + ... βn*(Clinical parameter or disease parameter or ionizing radiation treatment parameter (n) with n superior or equal to 2) + γ). The baseline hazard corresponds to the hazard of experiencing the event (PSE) when all covariates are zero. In one embodiment, the value entered for a given parameter in the formula hereinabove is 0 if said parameter is absent and 1 if said parameter is present (such as, for example, for the presence or absence of a comorbidity). In another embodiment, the value entered for a given parameter in the formula hereinabove corresponds to the measured value of said parameter. This embodiment applies to parameters measured as continued data, such as, for example, CTV, CTV1 and CTV2.

In one embodiment, Hazard (experiencing a radiation-induced side effect) = 1 - a^exp(α1* RILA + β1*(patient with class 1 urinary toxicity [0=no; 1=yes]) + β2*(patient with class 2 urinary toxicity [0=no; 1=yes])+ β3*(patient with class 3 urinary toxicity [0=no; 1=yes]) + β4*(patient with class 4 urinary toxicity [0=no; 1 =yes]) + γ).

In one embodiment, Hazard (experiencing a radiation-induced side effect) =1 - a^exp($\alpha$1* RILA + $\beta$1*(patient with class 1 urinary toxicity [0=no; 1=yes]) + $\beta$2*(patient with class 2 urinary toxicity [0=no; 1=yes]) + $\gamma$).

In one embodiment, Hazard (experiencing a radiation-induced side effect) =1 - a^exp($\alpha$1* RILA + $\beta$1*(patient with class 1 urinary toxicity [0=no; 1=yes]) + $\beta$2*(patient with class 2 urinary toxicity [0=no; 1=yes]) + $\beta$3*(patient with tumor T1B class [0=no; 1=yes]) + $\beta$4*(patient with tumor T1C class [0=no; 1=yes]) + $\beta$5*(patient with tumor T2A class [0=no; 1=yes]) + $\beta$6*(patient with tumor T2B class [0=no; 1=yes]) + $\beta$7*(patient with tumor T2C class [0=no; 1=yes]) + $\beta$8*(patient with tumor T3 class [0=no; 1=yes]) + $\beta$9*(patient with tumor T3A class [0=no; 1=yes]) + $\gamma$).

The $\gamma$ constant thus corresponds to the pondered sum of the average values in the population and the formula thus allows to detect the variation, for a patient, with regards to the mean of the data in the population. Thus, the risk or probability detected by the function is relative with regards to the population.

In one embodiment, Hazard (experiencing a radiation-induced side effect) may also be named risk to develop a radiation-induced side effect, for a subject.

In one embodiment, based on this multivariate Cox function, the skilled person would be able to introduce any additional relevant biochemical marker(s) and/or clinical, disease or ionizing radiation treatment parameter(s) to said multivariate Cox model.

In one embodiment, the different coefficients used for the values obtained for the different markers in the function of the invention, preferably in the multivariate Cox regression can be calculated through statistical analysis in a reference population of patients.

In one embodiment, the method of the invention thus comprises measuring an end-value, wherein said end-value is indicative of the risk of the subject to develop a radiation-induced side effects described hereinabove. In one embodiment, said risk is estimated taken into account all co-variables (biochemical markers and clinical parameters, disease parameters and/or ionizing radiation treatment parameters, which are combined in a mathematical function).

Therefore, in one embodiment, the "end-value" is the linear-predictor allowing the calculation of probability of experiencing a radiation-induced side effect for each subject.

In one embodiment, depending on the end-value obtained for a subject, it is possible to predict for said subject the risk of developing a radiation-induced side effect, during follow-up after ionizing radiation treatment, such as, for example, 3 months or 6, 12, 18, 24, 30 or 36 months after the end of the ionizing radiation treatment. The higher the end-value, the higher the risk of developing PSE.

In another embodiment, the choice of the optimal model of the invention is assessed by the Harrell's C-index for censored observations and is equal to the probability of concordance between two survival distributions (Harrell and Shih 2001). The C-index or concordance index quantifies the level of concordance between predicted probabilities and the actual chance of having the event of interest. In one embodiment, the method of the invention has a Harrell's C-index of at least about 0.5, preferably of at least about 0.55, more preferably of at least about 0.56, 0.57, 0.58 or more. In one embodiment, the method of the invention has a Harrell's C-index of at least about 0.585 or 0.590.

In the preferred embodiment, the choice of the optimal model of the invention is assessed by the Area Under the Receiving Operating Curve (AUROC). It is preferred when the formula has an AUROC higher than 0.63, more preferably equal or higher than 0.64, more preferably equal or higher than 0.65, more preferably equal or higher than 0.66, more preferably equal or higher than 0.67, more preferably equal or higher than 0.68, more preferably equal or higher than 0.69, most preferably equal or higher than 0.7.

In one embodiment, depending on the end-value obtained for a subject, it is possible to predict for said subject the risk of developing a radiation-induced side effect, during follow-up after ionizing radiation treatment, such as, for example, 3 months or 6, 12, 18, 24, 30 or 36 months (in particular 24 months) after the end of the ionizing radiation treatment.

By making a threshold vary from 0 to 1, it is possible to plot the AUROC (calculating the sensitivity and the specificity for each threshold). The choice of a threshold makes it possible to obtain a reference end-value that can be used by the physician to decide whether the patient has a "clinical" risk of developing PSE (if the end-value for the patient is higher than the reference end-value) or not (if the end-value for the patient is below the reference end-value). One can note that different reference end-values can be be selected, depending on whether one want to maximize the sensitivity (Se, detection of true positives), specificity (Sp, nondetection of true negatives), positive predictive value (PPV, probability that the patient is not a false positive), or negative predictive value (NPV, probability that the patient is not a false negative). Generally, it is interesting, for such methods, to maximize the NPV, in order to lower the risk of PSE for patients offered with a radiation treatment. Alternatively, selecting a reference end-value to maximaze the Se makes it possible to propose alternative treatment to patients with a risk of making PSE, but this would generally be balanced by a lower Sp. The method is very interesting to stratify (or classify) patients, so as to be able to propose a treatment that is adapted and specific to the patient. Thus, and as described below, depending on the risk of the patient, and generally on the risk of tumor recurrence, the physician will be able to propose a treatment that would maximaze the chances of cure, and minimize the secondary effects. Thus, the methods herein disclosed are of great interest in providing a help to the physician when investigating a clinical case. Consequently, even when obtaining an end-result and comparing it with a reference threshold, the physician shall also take into account the clinical and general context to be able to propose the treatment.

In one embodiment, the method of the invention comprises comparing the end-value obtained for a subject with a reference end-value.

As seen above, it is preferred when the end-value is compared to the reference value estimated by the model which discriminate patients with PSE and patients without PSE.

If the end-value is higher to the reference end-value, patients is at high level of risk to develop a PSE, in particular when the end-value is more than 10% higher than the reference end-value.

If the end-value is lower than the reference end-value, patients is at low level of risk to develop a PSE, especially when the end-value is more than 10% lower than the reference end-value.

In one embodiment, the method of the invention is computerized (or computer-implemented).

Another object of the present invention is thus a computer software for implementing the method of the invention.

In one embodiment, the method of the invention is implemented with a microprocessor comprising a software configured to calculate an end-value resulting from the combination of the measures of at least one biochemical marker (preferably RILA) and at least one clinical parameter, disease parameter and/or ionizing radiation treatment parameter (preferably neoadjuvant, concomitant and/or adjuvant hormonotherapy, tobacco smoking, at least one comorbidity and/or at least one ionizing radiation treatment parameter (in particular CTV, CTV1 and/or CTV2), more preferably neoadjuvant, concomitant and/or adjuvant hormonotherapy).

Another object of the present invention is directed to a system including a machine-readable memory, such as a computer and/or a calculator, and a processor configured to compute said mathematical function, in particular said multivariate Cox function, and provide the end-value for a given patient. This system may be dedicated to perform the method according to the invention.

In one embodiment, the method of the invention thus comprises:

- measuring at least one biochemical marker, preferably RILA and at least one clinical parameter, disease parameter or ionizing radiation treatment parameter, preferably neoadjuvant, concomitant and/or adjuvant hormonotherapy, tobacco smoking, at least one comorbidity and/or at least one ionizing radiation treatment parameter (in particular CTV, CTV1 and/or CTV2), more preferably neoadjuvant, concomitant and/or adjuvant hormonotherapy, for all subjects of a reference population;
- univariate analysis (estimation for each parameter one by one in order to select all significant parameters with p-value $\leq 0.2$) under a Cox regression model;
- multivariate analysis (estimation including all selected parameters by univariate analysis + adding optional non-significant parameters which are clinically relevant) under Cox regression model;
- selection of significant parameters and/or clinically relevant to obtain the final model whose linear predictor were extracted to estimate the risk (probability) to develop a radiation-induced side effect; linear predictor were integrated in a software;

According to one embodiment, the method of the invention comprises implementing the data obtained in step a) and in step b) to a computer or a calculator that will calculate the multivariate Cox regression and the risk of developing of a radiation-induced side effect. The data obtained by the physician is therefore more easily interpretable, and will allow for an improvement in the process for deciding the adapted patient care.

The invention also relates to a device for the prognosis of the risk of a patient to have PSE, comprising a first means, wherein the first means provides an end value by combining the parameters as indicated above through a mathematical function as disclosed herein.

The functions and markers to be implemented within this device are the ones disclosed in the present specification. As herein disclosed the device preferably makes it possible to obtain the end value using the functions disclosed above.

Another object of the present invention is a kit for collecting data of a subject to be further used for detecting the risk of developing of a radiation-induced side effect in said subject using the method of the present invention, wherein the kit comprises:

- a box/container and bag suited for biological transportation of a T cell containing sample, in particular a blood sample; and
- forms to be completed by the patient and/or the nurse and/or the physician, specifically designed and necessary to implement the method of the invention and provide the necessary information to be combined in the formula.

As an example, the forms may contain specific questions aimed at collecting information necessary to run the predictive analysis such as, age, whether the patient has undergone or will undergo an adjuvant treatment (chemotherapy or hormone therapy, for example), tobacco smoking habit, comorbidities, whether the patient has urinary toxicity, whether the patient has gastro-intestinal toxicity, whether the patient has diabetes, whether the patient takes anticoagulants, characteristics of the ionizing radiation treatment planned to be applied to the subject and date and time when the T cell containing sample was recovered.

Another object of the present invention is a kit for detecting the risk of developing a radiation-induced side effect, in a subject using the method of the present invention, wherein said kit comprises:

- reagents for determining the values of at least one biochemical marker according to the invention, preferably RILA;
- optionally means for collecting information on at least one clinical parameter, disease parameter or ionizing radiation treatment parameter according to the invention, such as a survey.

By "kit" is intended any manufacture (*e.g.*, a package or a container). The kit may be promoted, distributed, or sold as a unit for performing the methods of the present invention. Furthermore, any or all of the kit reagents may be provided within containers that protect them from the external environment, such as in sealed containers. The kits may also contain an instructional material describing the kit and methods for its use. Kits are also provided that are useful for various purposes. The label or instructional material may provide a description of the content of the kit as well as instructions for the intended use.

In one embodiment, the reagents for determining the values of at least one biochemical marker according to the invention correspond to some or all specific reagents required to run the RILA assay in an independent laboratory, where the irradiation of the sample may be run, for example, by a linear accelerator or a lab irradiator.

In one embodiment, the means for collecting information on at least one clinical parameter, disease parameter or ionizing radiation treatment parameter according to the invention correspond to specific forms to be completed by the patient and/or the nurse and/or the physician, specifically designed and required to run the method of the invention and the nomogram analysis. In a preferred embodiment, these forms may contain specific questions aimed at collecting information necessary to run the predictive analysis such as age, whether the patient has undergone or will undergo an adjuvant treatment (hormonotherapy, for example), tobacco smoking habit, comorbidities, whether the patient has urinary toxicity, whether the patient has gastro-intestinal toxicity, whether the patient has diabetes, whether the patient takes anticoagulants, parameters of the ionizing radiation treatment planned in the subject and date and time when the T cell containing sample was recovered.

In one embodiment, the kit for detecting the risk of developing radiation-induced side effects (*e.g.*, PSE as described hereinabove) in a subject comprises:

- some or all specific reagents required to run the RILA assay in an independent laboratory, where the irradiation of the sample may be run, for example, by a linear accelerator or a lab irradiator; and
- optionally specific forms to be completed by the patient and/or the nurse and/or the physician, specifically designed and required to run the method of the present invention.

Examples of specific reagents required to run the RILA assay include, but are not limited to, PBS, anti-human CD8-FITC antibodies and propidium iodide.

In one embodiment, determining the end-value for a subject will help the physician to adapt the dose and sequences of ionizing radiation treatment to the subject to limit the radiation-induced side effects, and optionally to adapt the treatment by replacing ionizing radiation treatment with other therapeutic treatments.

In one embodiment, the end-value (for example obtained with a multivariate Cox function) is used for the choice of a suitable treatment for the patient, such as an appropriate ionizing radiation treatment regimen, or surgery.

In one embodiment, the end-value is used for the choice of a suitable treatment for the patient or a suitable patient care, such as an appropriate ionizing radiation treatment dosage regimen, wherein:

- if the patient presents a risk to develop radiation-induced side-effects,the appropriate ionizing radiation treatment dosage regimen may be decreased for example by delivery of prostate hypofractionated treatment. Alternatively, in some embodiments, ionizing radiation treatment may be avoided completely (or postponed) for individuals with a high risk of toxicity, provided that an effective alternative exists. More specifically, for prostate cancer, surgery could be offered instead of ionizing radiation treatment for patients with high risk of toxicity. Patients with high-risk of toxicity could also be offered protontherapy treatment instead of conventional photontherapy treatment, or pelvic therapy, or MRI linac with reduced margin, or ionizing radiation treatment with rectal spacer or transponders (*e.g.*, with reduced margin), focal therapy or brachytherapy;
- if the patient presents low risk or no risk to develop radiation-induced side-effect, the patient might be offered a usual ionizing radiation treatment dosage regimen or a higher dose with modern techniques or ionizing radiation treatment in combination with systemic therapies with the aim of improving local control (*e.g.*, treatment with chemotherapy or radiosensitizers), or hypofractionated schedules (allowing decreased treatment duration) or dose escalation of ionizing radiation treatment (optionally with boost brachytherapy) or pelvic radiation therapy or stereotactic body radiation therapy.

As seen above, if the end-value is higher to the reference end-value, patients is at high level of risk to develop a PSE, in particular when the end-value is more than 10% higher than the reference end-value.

If the end-value is lower than the reference end-value, patients is at low level of risk to develop a PSE, especially when the end-value is more than 10% lower than the reference end-value.

Another object of the invention is a method for implementing an adapted patient care for a patient (preferably a prostate cancer patient), wherein said method comprises:

- assessing the risk for said patient to develop radiation-induced side-effects, using the *in vitro* method as described hereinabove;
- implementing an adapted patient care depending on the risk for the patient to develop radiation-induced side-effects.

In one embodiment, the patient presents a risk (*e.g.*, a high risk) to develop radiation-induced side-effect, and the adapted patient care is selected from a decreased ionizing radiation treatment dosage regimen (*e.g.*, delivery of prostate hypofractionated treatment), absence of ionizing radiation treatment (or postponement of ionizing radiation treatment), surgery, protontherapy treatment instead of conventional photontherapy treatment, MRI linac with reduced margin, ionizing radiation treatment with rectal spacer or transponders (*e.g.*, with reduced margin), pelvic therapy, focal therapy or brachytherapy.

In one embodiment, the patient presents a low risk or no risk to develop radiation-induced side-effect, and the adapted patient care is selected from a usual ionizing radiation treatment dosage regimen, or a higher dose of ionizing radiation treatment with modern techniques, dose escalation of ionizing radiation treatment (optionally with boost brachytherapy), pelvic ionizing radiation treatment, ionizing radiation treatment in combination with systemic therapies with the aim of improving local control (*e.g.*, treatment with chemotherapy or radiosensitizers), hypofractionated schedules (allowing decreased treatment duration) or stereotactic body ionizing radiation treatment.

In one embodiment, the patient presents a high risk of tumor recurrence and a low risk to develop radiation-induced side-effect, and the adapted patient care is selected from hypofractionation of ionizing radiation treatment and stereotactic body ionizing radiation treatment.

In one embodiment, the patient presents a low risk of tumor recurrence and a low risk to develop radiation-induced side-effect, and the adapted patient care is selected from dose escalation of ionizing radiation treatment (optionally with boost brachytherapy) and pelvic ionizing radiation treatment.

In one embodiment, the patient presents a high risk of tumor recurrence and a high risk to develop radiation-induced side-effect, and the adapted patient care is selected from focal therapy, brachytherapy, ionizing radiation treatment with rectal spacer or with transponders with reduced margin.

In one embodiment, the patient presents a low risk of tumor recurrence and a high risk to develop radiation-induced side-effect, and the adapted patient care is selected from surgery, ionizing radiation treatment with rectal spacer or with transponders, pelvic therapy or proton therapy.

Methods to assess the risk of tumor recurrence are well-known by the skilled artisan and include, without limitation, the use of the classification developed by D'Amico et al (JAMA, 1998, 280(11):969-74). The methods for determining / proposing a treatment are also described in Azria et al (Front Oncol. 2017 Apr 27;7:83).

**EXAMPLES**

The present invention is further illustrated by the following examples, which are part of the invention.

Example 1:

***Materials and methods***

*Patient analysis*

Analysis was performed among 383 prostate cancer patients treated by radiotherapy (*i.e.*, ionizing radiation treatment). Patients were followed with a median follow-up of 38.2 months. A specific questionnaire (Case Report Form) was filled out for each patient including general social demographics administrative data, risk factors, and at each visit, clinical and biological and treatment items, as described in **Table 1**. RILA and complete RT were performed for 352 patients before entering follow-up. 240 patients were followed for at least 36 months according to the protocol.

*Radiation-induced CD8 T-lymphocyte Apoptosis (RILA) Procedure*

The protocol was adapted from studies of Ozsahin et al. (Ozsahin, Crompton et al. 2005). Before radiotherapy (RT), one blood sample was collected from each patient in a 5-mL heparinized tube. 200 μL of blood was aliquoted into a 6-well plate. All tests were carried out in triplicate for both 0 and 8 Gy. Irradiations (single dose of 8 Gy in a 25 cm × 25 cm field size at a dose rate of 1 Gy/min) were delivered after 24 h (H24) using a linear accelerator (2100 EX, 200 UM/min, Varian, US). Control cells were removed from the incubator and placed for the same period of time under the Linac but without radiation treatment. After irradiation, the flasks were immediately incubated at 37°C (5% $CO_2$). After a further forty-eight hours (H72), cells were labeled with an anti-human CD8-FITC antibody (10 μL/tests, Becton Dickinson, USA). After addition of lysis buffer (Becton Dickinson, USA), propidium iodide (Sigma, France) and RNAse (Qiagen, France) were added to each tube and the samples were prepared for flow cytometry (FACS).

RILA assay commonly comprises the following steps:

    a) cell culture of blood samples
    b) sample irradiation at 8Gy using a linear accelerator, and
    c) sample labelling for evaluation of T-lymphocytes apoptosis, in particular with FACS analysis.

The person skilled in the art may use classical methods to manage the RILA assay. The evaluation of T-lymphocytes apoptosis at the step c) may use alternative methods such as dosage of Anexin 5, dosage of caspases or FACS analysis, preferably FACS analysis.

Preferred protocols are as follows:

Cell culture of blood samples:

- one blood sample is collected from each patient in heparinized tubes and a volume of blood is aliquoted into well plate;
- The Assay is generally carried out in triplicate (0 Gy and 8 Gy).
- A culture medium is added (ex: add 2 ml of medium RPMI1640 (Gibco), supplemented with 20% FCS) per well in 6 well tissue culture plates.
- Place the tissue culture plates in culture for 24 hours at 37°C, 5% CO2.

Sample irradiation at 8Gy using a linear accelerator:

- After 15 to 30 hours of culture, in particular after 24h, the plates are safely transported to the irradiator and handle to a radiation technologist, for applying an irradiation of 8Gy with a dose rate of 1Gy/min.

In the preferred embodiment, the conditions of the irradiation are:

Dose rate = 1Gy/min
Energy = 6MV
Depth = 15 mm polystyrene on cells.
DSP (Distance between Source and Plate) = 145cm
Field = 25 * 25cm² collimator.

Linear accelerator (machine output: 200UM/mn): 200UM for 1Gy: 1600UM for 8Gy.

- Control cells are removed from the incubator and placed for the same period of time but without radiation treatment.
- plates are incubated for 40 to 50 hours at 37°C, 5% CO2.

Sample labelling for evaluation of T-lymphocytes apoptosis, in particular with FACS analysis:

- after 40 to 50 hours incubation, in particular 48h of incubation, the plates are removed from the incubator and placed at room temperature;
- the content of each well is transferred to pre-labeled centrifuges tubes, and after centrifugation (ex: 5mn at 1450rpm), the content is labeled with anti-human CD4 and/or CD8-FITC antibody, preferably CD8-FITC antibody;
- after addition of lysis buffer, reagents are added to each tube for evaluating lymphocytes apoptosis according to usual methods known from the man skilled in the art, in particular according to flow cytometry method (FACS) with propidium iodide and RNase A as reagents.

*Preparation and Delivery of Radiotherapy*

Patients underwent CT-based virtual simulation (CT Simulator, General Electric, Cleveland, OH) with 2.5 mm thick slices obtained at 2.5 mm intervals in the supine position. A small flexible rectal tube was inserted to evacuate flatus, and then removed. Intravenous contrast was used in all patients to permit better delineation of pelvic lymph nodes and bladder. Patients were positioned with knee and feet support (Sinmed, The Netherlands) but no custom immobilization device was used. The isocenter was set in the middle of the prostate using our virtual simulation console (AdvantageSim, General Electric, Cleveland, OH) by the treating physician immediately after the first scan, while the patient waited in the treatment position on the scan table. The isocenter was then tattooed on the patient skin using our scan room mobile lasers (LAP Dorado CT-4).

Structures were manually contoured on the CT scan. When pelvic irradiation was indicated, the clinical target volume (CTV) for the lymph nodes included the obturator, internal iliac, external (excluding the artery) iliac and sacral vessels plus a circumferential 7 mm margin in accordance with the RTOG consensus guidelines for contouring.

The planning target volumes (PTV) 1 was defined as the prostate and seminal vesicles (proximal part in the absence of invasion) plus a 1 cm margin in all directions except posteriorly (5 mm), plus the CTV for lymph nodes and a 7 mm set-up margin when indicated.

In case of a post-prostatectomy radiotherapy, the planning target volumes (PTV) 1 was defined as the prostate bed plus a 1 cm margin in all directions except posteriorly (5 mm), plus the CTV for lymph nodes and a 7 mm set-up margin when indicated.

The PTV2 consisted of the prostate gland only or the prostate gland plus invaded seminal vesicles, with a 1 cm (5 mm posteriorly) margin.

In case of a post-prostatectomy radiotherapy, the PTV2 consisted of the prostate bed only with a 1 cm (5 mm posteriorly) margin.

The bladder was contoured in its entirety. The rectum was contoured as a whole organ but starting 2 cm above and below the CTV. Femoral heads were drawn from the top of the acetabulum to the small trochanter inferiorly. Small bowel was determined in all slices where the PTV was apparent. To take interfractional bowel motion into account, the small bowel was delineated as a whole pelvic and abdominal cavity excluding bones, muscle and other organs at risk (OAR), rather than contoured as individual bowel loops. For bowel, rectum and bladder, a second volume was created and defined as the considered organ minus the PTV (bowel - PTV, rectum - PTV, bladder - PTV) to avoid hot spots and improve optimization.

In case of prostate in place, the prescribed doses to the PTV1 and PTV2 were 54 Gy and 74-80 Gy in 37-40 daily fractions, respectively.

In case of radiotherapy post-prostatectomy, the prescribed doses to the PTV1 and PTV2 were 54 Gy and 66-70 Gy in 37 daily fractions, respectively.

RapidArc® plans were performed using the Eclipse software version 8.9.08 (Helios, Varian, Palo Alto, CA). A maximum dose rate of 600 MU/min and 18 MV photon beams were selected. RapidArc with 1 arc (RA1) corresponded to a single 360° rotation, and RapidArc with 2 arcs (RA2) to two coplanar arcs of 360° sharing the same isocenter and optimized independently and simultaneously. These two arcs were delivered with opposite rotation (clock and counter-clock) so that off-treatment between the two beams was minimized to about 25 seconds. For RA1, field size and collimator rotation were determined by the automatic tool from Eclipse to encompass the PTV. We controlled that the collimator was rotated to a value different from zero in order to avoid the tongue-and-groove effect. For RA2, the first arc was similar to that defined in the RA1 process except for the rotation of the collimator, which was 360-X for the second arc (X corresponded to the rotation of the collimator of the first arc).

Optimization process was undertaken by decreasing as much as possible the dose to OAR without altering PTV coverage, and the results were improved by modifying constraints and priority factors. These parameters were modified with regard to the DVH results for each patient. We always checked that a good PTV2 coverage by the 74-80 Gy isodose was obtained, 95% of the PTV receiving at least 95% of the prescribed dose. Regarding rectal dose, the maximal dose allowed was 74-80 Gy, the volume receiving 72 Gy (V72) should remain less than 25 % and the V60 less than 50 %. For bladder, the maximal dose was 74 Gy, V70 should remain less than 50 % and V75 less than 25 %. The maximal dose to the femoral heads was 55 Gy and V50 was less than 5 %. The maximal dose allowed for small bowel was 34 Gy, with less than 450 cc receiving 30 Gy or more and less than 200 cc receiving 40 Gy or more.

VMAT plans with one arc or 2 arcs were optimized to try to achieve the best results with the possibility offered by the software.

*Adjuvant Systemic Therapies*

The LHRH agonist or GnRH antagonist, was administered subcutaneously every 4 weeks or every 3 months or every 6 months starting before or during radiotherapy and continued for 4 months to 3 years; In some patients, a steroidal antiandrogen was given orally for 1 month starting a week before LHRH agonist to prevent the flare that results from the testosterone surge occurring after LHRH agonist administration.

*End-point Assessments: identification of biomarkers and relevant covariables as prognostic factors of PLE on the reference population*

The primary objective was the predictive role of RILA in radiation-induced grade ≥2 radiation-induced side effects, either acute or late (defined as dysuria, pollakiuria, increased urinary frequency, decreased urine stream, burning urination, bleedings, urinary incontinence, mictional or urinary impetuosity, nocturia, hematuria, urinary pain, diarrhea, constipation, rectal syndrome, functional proctitis and/or anusitis, physical proctitis and/or anusitis, radiation proctitis, ano-proctitis, rectal bleeding, anal bleeding, rectal or anal pain, sexual impotence, erectile dysfunction, ejaculation dysfunction and pelvic neuropathy).

Toxicity evaluations were performed at baseline, every week during RT, one, three and six months after the last RT fraction, every 6 months up to month 36. Each evaluation was assessed by the physicians blinded for RILA. The severe grade ≥2 PLE observed during the follow-up after RT was considered as the primary endpoint. The other severe late effects (lung, cardiac, skin, lung, for example) observed from 12 weeks to 3 years post RT and the most severe acute side effects observed from the start of RT to 12 weeks post RT were considered as the secondary endpoints. Toxicities were evaluated using all the possible definitions described by (Trotti, Colevas et al. 2003).

All endpoints were defined as the interval between the start of RT and following the first events: death for Overall Survival (OS), local or distant recurrence or death for Relapse-Free Survival (RFS), grade ≥2 PLE for Complication-Free Survival (CFS), and first event of RFS and C-FS for Complication-Relapse Free Survival (C-RFS) (Peto, Pike et al. 1977). Censoring patients were patients alive at the last follow-up visit for OS, patients alive and without relapse for RFS, patients alive who never experienced a grade ≥2 PLE for C-FS and patients alive who never experienced grade ≥2 PLE or relapse for C-RFS.

*Sample Size Calculation and Statistical Analysis*

To test the prognostic value of RILA rate on the occurrence of PLE, we started from the results of our preliminary study (Azria, Gourgou et al. 2004) and Zelefsky study (Zelefsky et al., 2002). Details are presented in the following protocol. Briefly, based on $\sigma2 = 0.54$, an estimated gastrointestinal complication rate of $\psi = 5\%$ and genitourinary complication rate of $\psi = 10\%$, with a two-sided $\alpha$ error of 0.05 and a $\beta$ error of 0.05 (power=0.95), 368 patients had to be included.

$\sigma2$ is the variance of the studied variable (logCD8) and $\psi$ is the rate of complication/toxicity expected events.

The cumulative incidences of complications as a function of the prognostic variables were calculated using a non-parametric model (Pepe and Mori 1993). The main statistical procedure included a multivariate analysis using the Fine et al. model of competing risks (Fine 2001) for the assessment of the impact of RILA rate on the occurrence of PLE in the presence of other events (such as relapse or death) that are considered as competing risk events in this pathology. For multivariate analysis, selected factors were the baseline parameters with a p-value (statistical significance) less than 0.20 in univariate analysis. Final model was defined using backward stepwise selection (p<0.15) and a step by step method was used to include only the significant parameters (p<0.05) or clinically relevant and/or (p<0.10).

Data were summarized by frequency and percentage for categorical variables and by median and range for continuous variables. Absolute changes in RILA counts before and after irradiation were evaluated as continuous and categorical variables. Three categories were constructed around the 33% quantiles = tertile (≤15, ]15-24], and >24) and then merged in two categories (≤15 and >15).

OS, RFS, C-FS and C-RFS rates were estimated by the Kaplan-Meier method (Kaplan and Meier 1958). Ninety-five percent confidence intervals (95%CI) were also determined.

Univariate analysis and multivariate analysis were performed using the Cox proportional hazard's regression model (Cox, et al. 1984) to estimate the hazard ratio including baseline characteristics and treatment parameters. Comparisons were performed using the log-rank test for univariate analysis. Independent effects were evaluated from the likelihood ratio statistics.

Impact of RILA on complication -relapse-free survival (C-RFS) was assessed. The cumulative incidence of PLE and relapse or death were estimated from a competing risk model using estimates obtained from the cause-specific hazard functions and the composite RFS and C-FS distribution (Arriagada, Rutqvist et al. 1992) and compared using Gray's test.

Median follow-up was estimated with the inverse Kaplan-Meier method. A p-value less than 0.05 was considered as significant. All statistical tests were two-sided. Stata was used for all statistical analyses (version 13.0) and the SAS macro %cif was used for Gray's test.

To complement analysis, receiver-operator characteristic (ROC) curve analyses for RILA were performed to identify patients who experienced at least a grade 2 PLE during the follow-up (Kramar, Faraggi et al. 2001). The empirical areas under the ROC curves (AUC) and the respective 95%CI were used for RILA to determine the sensitivity, specificity, positive predictive value (PPV), and negative predictive value (NPV).

### Results

*Descriptive statistical analysis for modelling*

The analysis was performed on selected data from studies for all patients observing independent variables, as presented in **Table 1** below.

Table 1

| | N=352 ('reference population') |
|---|---|
| **Age (Year)** | |
| Median (range) | 69.00 (49.00:88.00) |
| **Weight (kg)** | **n = 339** |
| Median (range) | 79.0 (58.0 :160.0) |
| **Baseline RILA** | |
| Median (range) | 19.7 (1.6:68.8) |
| **PSA dosing** | **n=335** |
| Median (range) | 8.4 (0.00:86.3) |
| **Gleason** | **n=342** |
| 5-6<br>7<br>8-9-10 | 116<br>176<br>50 |
| **High blood pressure** | |
| No<br>Yes | 201<br>151 |
| **Diabetes** | |
| No<br>Yes | 301<br>51 |
| **Angina pectoris** | |
| No<br>Yes | 342<br>10 |
| **Heart failure** | |
| No<br>Yes | 328<br>24 |
| **Chronic hepatitis** | |
| No<br>Yes | 345<br>7 |
| **Respiratory failure** | |
| No<br>Yes | 343<br>9 |
| **Arteritis** | |
| No<br>Yes | 345<br>7 |

(continued)

| Hypercholesterolem ia | |
|---|---|
| No | 282 |
| Yes | 70 |
| **Tobacco smoking** | **n=337** |
| Non smoker | 140 |
| Ex-smoker or Smoker | 197 |
| **Adjuvant Hormonotherapy** | |
| No | 162 |
| Yes | 190 |
| Agonists LHRH<br>No<br>Yes | <br>5<br>185 |
| Antiandrogens<br>No<br>Yes | <br>132<br>56 |
| **Anticoagulants** | |
| No | 256 |
| Yes | 96 |
| **Sexual disorders** | |
| Grade 0-1 | 299 |
| Grade 2-3-4 | 25 |
| **Urinary disorders** | |
| Grade 0-1 | 340 |
| Grade 2-3-4 | 11 |
| **Digestive disorders** | |
| Grade 0-1 | 347 |
| Grade 2-3-4 | 4 |
| **At least one disorder (among sexual, urinary and digestive disorders)** | |
| Grade 0-1 | 288 |
| Grade 2-3-4 | 63 |

According to the CTCAE V3.0 (Trotti, Colevas et al. 2003), toxicity was graded according to severity of symptoms.

The main endpoint was the identification of probability to develop a PLE during 3 years follow-up.

The first stage consisted in identification of prognostic factors of development of PLE using univariate analysis and using the log rank test.

The second stage consisted in analysis of multivariate Cox proportional hazard model to assess the independent parameters for the prediction of the occurrence of PLE and to estimate the effect size defined as Hazard ratio (HR).

The best model was selected using the highest AUROC.

Results show that the measure and mathematical combination of RILA, urinary disorders and optionally at least one other parameter in a COX model allows increasing the accuracy of prediction of the development of radiation-induced side effects as compared to the use of RILA alone.

Moreover, the pronostic performance of the value of RILA was assessed by sensitivity (Se), specificity (Sp), positive and negative predictive values (PPV, NPV) using the time-dependent receiver operating characteristic (AUROC). This method was used to build a categorical variable of RILA which maximize the diagnosis parameter as Se, Sp, PPV, NPV with the following hypothesis :

$$Se(t)=P(Xi>c \mid Ti \leq t)$$

$$Sp(t)=P(Xi \leq c \mid Ti>t)$$

where Ti = Time of Toxicity of patient i

For such prognosis of a responding phenotype, a responding condition or test outcome is considered a positive result, while a non-responding condition or test outcome is considered a negative result. True and false positive results, NPV, PPV, specificity, sensitivity are defined and calculated as follows (Table 2):

**Table 2**

| | | Condition (responding) | |
|---|---|---|---|
| | | *Positive* | *Negative* |
| **Test outcome (responding)** | *Positive* | True Positive (TP) | False positive (FP) |
| | *Negative* | False negative (FN) | True negative (TN) |
| PPV = TP / (TP+FP)<br>NPV = TN / (TN+FN)<br>Sp = TN / (TN+FP)<br>Se = TP / (TP+FN) | | | |

"ROC" or "ROC curve" is a tool for diagnostic/prognostic test evaluation, wherein the true positive rate (Sensitivity) is plotted in function of the false positive rate (1-Specificity) for different cut-off points of a parameter after classification of patients. Each point on the ROC curve represents a sensitivity/specificity pair corresponding to a particular decision threshold (from 0 to 1). The area under the ROC curve (AUC) is a measure of how well a parameter can distinguish between two diagnostic groups (diseased/normal). The accuracy of the test depends on how well the test separates the group being tested into those with and without the disease in question. Accuracy is measured by the area under the ROC curve. An area of 1 represents a perfect test; an area of 0.5 represents a worthless test.

It is usually acknowledged that a ROC area under the curve (AUROC) has a value superior to 0.7 is a good predictive curve for diagnosis, although an AUROC higher than 0.63 also indicates a function with a relatively good quality. The ROC curve has to be acknowledged as a curve allowing prediction of the diagnosis quality of the method.

### Example 3. Performance of RILA alone

In this example, RILA alone is studied for its prognosis ability to assess the risk of occurrence of adverse event in prostate cancer patients within two years after ionizing radiation treatment.

An AUROC of 0.59 was obtained, which indicates that RILA by itself is not sufficient as a good prognosis test for assessing the risk of occurrence of PSE.

### Example 4. Combining RILA with parameters disclosed in WO2018041960

WO2018041960 discloses a diagnosis method and a calculator for predicting the risk of developing a breast late effect (BLE) combining using Radiation Induced late effect using T-Lymphocyte Apoptosis (RILA) and clinical parameters, in particular tobacco smoking habits and adjuvant hormonotherapy.

The value of such combination has been tested for PSE. The AUROC obtained is 0.57, thereby indicating that a model developed using these criteria is very poor in prostate and has little predictive value.

### Example 5. Combining RILA with other parameters

Multiple Cox functions were generated, combining RILA with presence of urinary disorders (patients classified according to the CTCAE grade of the toxicity) in combination with other parameters. The data showed that combining RILA with presence of urinary disorders is found in all combinations that are of good prognosis quality. Further using the tumor T grade (TNM classification) also led to functions with good prognosis value. Adding other parameters (such as presence of diabetes, age of the patient, presence of gastro-intestinal disorders, intake of anticoagulants...) also made it possible to obtain functions with good prognosis value, as attested by AUROC higher than 0.63, or even higher than 0.70.

This example illustrates various embodiments of the invention. all functions herein disclosed are also subject of the invention. These functions are quantitative, i.e., the higher the end-result, the higher the relative risk of having PSE within 24 months.

As an illustration, RILA, presence of urinary toxicities, and tumor T grade were combined in a function F1:

$$F1 = 1 - (1 - a)^{\exp(f1)}$$

With f1 = α*RILA + β1*tumt1B + β2*tumt1C + β3*tumt2A + β4*tumt2B + β5*tumt2C + β6*tumt3 + β7*tumt3A + β8*tumt3B + β9*URI1 + β10*URI2 + γ

In this function f1

RILA represents the percentage of apoptotic lymphocytes as defined by RILA (difference between apoptosis of the irradiated lymphocytes and the apoptosis of non-irradiated lymphocytes)
tumt1B has value 1 if the tumor T grade of the patient is 1B and 0 if not
tumt1C has value 1 if the tumor T grade of the patient is 1C and 0 if not
tumt2A has value 1 if the tumor T grade of the patient is 2A and 0 if not
tumt2B has value 1 if the tumor T grade of the patient is 2B and 0 if not
tumt2C has value 1 if the tumor T grade of the patient is 2C and 0 if not
tumt3 has value 1 if the tumor T grade of the patient is 3 and 0 if not
tumt3A has value 1 if the tumor T grade of the patient is 3A and 0 if not
tumt3B has value 1 if the tumor T grade of the patient is 3B and 0 if not
URI1 has value 1 if the urinary toxicity is of grade 1 and 0 if not
URI2 has value 1 if the urinary toxicity is of grade 2 and 0 if not.

In function F1,
$0.10 \leq a \leq 0.2$, more preferably $0.125 \leq a \leq 0.175$, in particular a= 0.149
$-0.02 \leq \alpha \leq -0.01$, more preferably $-0.0175 \leq \alpha \leq -0.0125$, in particular $\alpha$ =-0.01577
$0.55 \leq \beta1 \leq 0.75$, more preferably $0.6 \leq \beta1 \leq 0.7$, in particular $\beta1$ = 0.64827
$-1.8 \leq \beta2 \leq -1.6$ , more preferably $-1.75 \leq \beta2 \leq -1.65$, in particular $\beta2$ = -1.7171
$-1.8 \leq \beta3 \leq -1.6$, more preferably $-1.75 \leq \beta3 \leq -1.65$, in particular $\beta3$ = -1.6999
$-1.9 \leq \beta4 \leq -1.6$, more preferably $-1.8 \leq \beta4 \leq -1.7$, in particular $\beta4$ = -1.7449
$-19.9 \leq \beta5 \leq -19.6$, more preferably $-19.8 \leq \beta5 \leq -19.7$, in particular $\beta5$ =-19.7449
$-19.3 \leq \beta6 \leq -19$, more preferably $-19.2 \leq \beta6 \leq -19.1$, in particular $\beta6$ =-19.14261
$-1.8 \leq \beta7 \leq -1.6$, more preferably $-1.75 \leq \beta7 \leq -1.65$, in particular $\beta7$ =-1.72674
$0.65 \leq \beta8 \leq 0.85$, more preferably $0.7 \leq \beta8 \leq 0.8$, in particular $\beta8$ = 0.77055
$0.75 \leq \beta9 \leq 0.95$, more preferably $0.8 \leq \beta9 \leq 0.9$, in particular $\beta9$ = 0.85974
$0.6 \leq \beta10 \leq 0.8$, more preferably $0.65 \leq \beta10 \leq 0.75$, in particular $\beta10$ = 0.71377
$0.7 \leq \gamma \leq 0.9$ , more preferably $0.75 \leq \gamma \leq 0.85$, in particular $\gamma$ = 0.80763
The values have been rounded to the third or fifth decimal.
In particular,

$$F1 = 1 - (1 - 0.149)^{\exp(f1)}$$

with

f1 = -0.01577*RILA + 0.64827*tumt1B - 1.7171*tumt1C - 1.6999*tumt2A - 1.7449*tumt2B - 19.7449*tumt2C - 19.14261*tumt3 - 1.72674*tumt3A + 0.77055*tumt3B + 0.85974*URI1 + 0.71377*URI2 + 0.80763.

This function has an AUROC of 0.705, therefore being of good prognosis quality. With a cut-off (reference end-value) of 0.053, the sensitivity is 0.701, specificity is 0.669, positive predictive value is 0.511 and negative predictive value is 0.820. This function with this cut-off is thus appropriate to identify the patients with low risk of having PSE.

It is to be noted that another cut-off can be determined with the physician, should there be a wish to particularly improve one or the other parameter (Sp, Se, PPV or NPV).

Another model was developed with RILA, presence of urinary toxicities, tumor T grade and presence/absence of diabetes (comorbidity) combined in a function F2:

$$F2 = 1 - (1 - a)^{\exp(f2)}$$

with f2 = α*RILA + β1*tumt1B + β2*tumt1C + β3*tumt2A + β4*tumt2B + β5*tumt2C + β6*tumt3 + β7*tumt3A + β8*tumt3B + β9*URI1 + β10*URI2 + β11*DIAB + γ

In this function f2

RILA represents the percentage of apoptotic lymphocytes as defined by RILA
tumt1B has value 1 if the tumor T grade of the patient is 1B and 0 if not
tumt1C has value 1 if the tumor T grade of the patient is 1C and 0 if not
tumt2A has value 1 if the tumor T grade of the patient is 2A and 0 if not
tumt2B has value 1 if the tumor T grade of the patient is 2B and 0 if not
tumt2C has value 1 if the tumor T grade of the patient is 2C and 0 if not
tumt3 has value 1 if the tumor T grade of the patient is 3 and 0 if not
tumt3A has value 1 if the tumor T grade of the patient is 3A and 0 if not
tumt3B has value 1 if the tumor T grade of the patient is 3B and 0 if not
URI1 has value 1 if the urinary toxicity is of grade 1 and 0 if not
URI2 has value 1 if the urinary toxicity is of grade 2 and 0 if not.
DIAB has value 1 if the patient has diabetes and 0 if not

In function F2,
$0.10 \leq a \leq 0.2$, more preferably $0.125 \leq a \leq 0.175$, in particular a= 0.147
$-0.02 \leq \alpha \leq -0.01$, more preferably $-0.0175 \leq \alpha \leq -0.0125$, in particular $\alpha$ = -0. 01455
$0.10 \leq \beta1 \leq 0.35$, more preferably $0.18 \leq \beta1 \leq 0.3$, in particular $\beta1$ = 0.23626
$-2.25 \leq \beta2 \leq -1.95$, more preferably $-2.15 \leq \beta2 \leq -2.05$, in particular $\beta2$ =-2.10091
$-2.2 \leq \beta3 \leq -1.95$, more preferably $-2.15 \leq \beta3 \leq -2.0$, in particular $\beta3$ =-2.07048
$-2.2 \leq \beta4 \leq -1.95$, more preferably $-2.15 \leq \beta4 \leq -2.0$, in particular $\beta4$ =-2.08434
$-20.35 \leq \beta5 \leq -20.05$, more preferably $-20.25 \leq \beta5 \leq -20.15$, in particular $\beta5$ =-20.20423
$-19.7 \leq \beta6 \leq -19.4$, more preferably $-19.6 \leq \beta6 \leq -19.5$, in particular $\beta6$ =-19.55779
$-2.15 \leq \beta7 \leq -1.95$, more preferably $-2.10 \leq \beta7 \leq -2.0$, in particular $\beta7$ =-2.04109
$-1.35 \leq \beta8 \leq -1.05$, more preferably $-1.25 \leq \beta8 \leq -1.15$, in particular $\beta8$ =-1.19540
$0.75 \leq \beta9 \leq 1.05$, more preferably $0.8 \leq \beta9 \leq 1.0$, in particular $\beta9$ = 0.89858
$0.55 \leq \beta10 \leq 0.75$, more preferably $0.6 \leq \beta10 \leq 0.70$, in particular $\beta10$ = 0.67333
$0.45 \leq \beta11 \leq 0.65$, more preferably $0.5 \leq \beta11 \leq 0.6$, in particular $\beta11$ =-0.56124
$0.8 \leq \gamma \leq 1.0$ , more preferably $0.85 \leq \gamma \leq 0.95$, in particular $\gamma$ = 0.90876
The values have been rounded to the third or fifth decimal.
In particular,

$$F2 = 1 - (1 - 0.147)^{\exp(f2)}$$

with f2 = -0.01455*RILA + 0. 23626*tumt1B - 2.10091*tumt1C - 2.07048*tumt2A - 2.08434*tumt2B - 20.20423*tumt2C - 19.55779*tumt3 - 2.04109*tumt3A - 1.19540*tumt3B + 0.89858*URI1 + 0.67333*URI2 - 0.56124*DIAB + 0.90876.

This function has an AUROC of 0.719, therefore being of good prognosis quality. With a cut-off (reference end-value) of 0.040, the sensitivity is 0.731, specificity is 0.676, positive predictive value is 0.527 and negative predictive value is 0.836. This function with this cut-off is thus appropriate to identify the patients with low risk of having PSE.

It is to be noted that another cut-off can be determined with the physician, should there be a wish to particularly improve one or the other parameter (Sp, Se, PPV or NPV).

Another model was developed with RILA, presence of urinary toxicities, tumor T grade and level of PSA combined in a function F3:

$$F3 = 1 - (1-a)^{\exp(f3)}$$

with $f3 = \alpha*RILA + \beta1*tumt1B + \beta2*tumt1C + \beta3*tumt2A + \beta4*tumt2B + \beta5*tumt2C + \beta6*tumt3 + \beta7*tumt3A + \beta8*tumt3B + \beta9*URI1 + \beta10*URI2 + \beta11*PSA + \gamma$$

In this function f3

RILA represents the percentage of apoptotic lymphocytes as defined by RILA
tumt1B has value 1 if the tumor T grade of the patient is 1B and 0 if not
tumt1C has value 1 if the tumor T grade of the patient is 1C and 0 if not
tumt2A has value 1 if the tumor T grade of the patient is 2A and 0 if not
tumt2B has value 1 if the tumor T grade of the patient is 2B and 0 if not
tumt2C has value 1 if the tumor T grade of the patient is 2C and 0 if not
tumt3 has value 1 if the tumor T grade of the patient is 3 and 0 if not
tumt3A has value 1 if the tumor T grade of the patient is 3A and 0 if not
tumt3B has value 1 if the tumor T grade of the patient is 3B and 0 if not
URI1 has value 1 if the urinary toxicity is of grade 1 and 0 if not
URI2 has value 1 if the urinary toxicity is of grade 2 and 0 if not.
PSA is the measured circulating PSA (in ng/ml)

In functions F3 and f3,
$0.10 \leq a \leq 0.18$, more preferably $0.115 \leq a \leq 0.155$, in particular a= 0.134
$-0.02 \leq \alpha \leq -0.01$, more preferably $-0.0175 \leq \alpha \leq -0.0125$, in particular $\alpha$ =-0.01544
$0.10 \leq \beta1 \leq 0.35$, more preferably $0.18 \leq \beta1 \leq 0.3$, in particular $\beta1 = 0.54215$
$-2.3 \leq \beta2 \leq -1.7$ , more preferably $-2.1 \leq \beta2 \leq -1.9$, in particular $\beta2 = -1.99374$
$-2.2 \leq \beta3 \leq -1.6$, more preferably $-2.0 \leq \beta3 \leq -1.8$, in particular $\beta3 = -1.89521$
$-2.15 \leq \beta4 \leq -1.55$, more preferably $-1.95 \leq \beta4 \leq -1.75$, in particular $\beta4$ =-1.84661
$-20.3 \leq \beta5 \leq -19.7$, more preferably $-20.1 \leq \beta5 \leq -19.9$, in particular $\beta5$ =-20.01361
$-19.7 \leq \beta6 \leq -19.1$, more preferably $-19.5 \leq \beta6 \leq -19.3$, in particular $\beta6$ =-19.39313
$-2.65 \leq \beta7 \leq -2.15$, more preferably $-2.5 \leq \beta7 \leq -2.3$, in particular $\beta7$ =-2.39313
$-1.3 \leq \beta8 \leq -0.7$, more preferably $-1.1 \leq \beta8 \leq -0.8$, in particular $\beta8 = -1.02025$
$0.7 \leq \beta9 \leq 1.3$, more preferably $0.9 \leq \beta9 \leq 1.1$, in particular $\beta9 = 1.00285$
$0.55 \leq \beta10 \leq 0.95$, more preferably $0.65 \leq \beta10 \leq 0.85$, in particular $\beta10 = 0.77178$
$0.005 \leq \beta11 \leq 0.035$, more preferably $0.01 \leq \beta11 \leq 0.03$, in particular $\beta11$ =-0.02046
$0.75 \leq \gamma \leq 1.35$ , more preferably $0.95 \leq \gamma \leq 1.15$, in particular $\gamma = 1.07431$
The values have been rounded to the third or fifth decimal.
In particular,

$$F3 = 1 - (1-0.134)^{\exp(f3)}$$

with f3 = -0.01544*RILA + 0.54215*tumt1B - 1.99374*tumt1C - 1.89521*tumt2A – 1.84661*tumt2B - 20.01361*tumt2C - 19.39313*tumt3 - 2.39313*tumt3A - 1.02025*tumt3B + 1. 00285*URI1 + 0. 77178*URI2 - 0.02045*PSA + 1.07431.

This function has an AUROC of 0.714, therefore being of good prognosis quality. With a cut-off (reference end-value) of 0.041, the sensitivity is 0.726, specificity is 0.605, positive predictive value is 0.469 and negative predictive value is 0.821. This function with this cut-off is thus appropriate to identify the patients with low risk of having PSE.

It is to be noted that another cut-off can be determined with the physician, should there be a wish to particularly improve one or the other parameter (Sp, Se, PPV or NPV).

Other functions were developed combining RILA, presence (and grade) of urinary toxicity, and other parameters (age, intake of anticoagulants, tumor T grade, tumor N grade, presence of gastro-intestinal toxicity, level of PSA). Various combinations of these parameters were used together with RILA and presence (and grade) of urinary toxicity.

The functions obtained presented AUROC comprised between 0.63 and 0.73, most of them having an AUROC higher than 0.7.

This example shows that using RILA and presence (and grade) of urinary toxicity in a mathematical function makes it possible to obtain a function that can be used for making prognosis of the occurrence of side effect after ionizing radiation treatment in patients with prostate cancer.

Other functions can be developed, using the methods disclosed above, and with other parameters added to the two herein disclosed.

**Claims**

1.  An *in vitro* method for assessing the risk of developing side effects after ionizing radiation treatment in a patient suffering from prostate cancer, comprising:

    a) measuring at least one biochemical marker in a sample of the patient, wherein said at least one biochemical marker is radiation induced T-lymphocytes apoptosis (RILA);
    b) determining the presence of urinary toxicity in the patient prior to application of ionizing radiation, and optionally of at least one other clinical parameter, disease parameter or ionizing radiation treatment parameter from the patient, and
    c) combining the value of the at least one biochemical marker measured in (a) and a value associated with the at least one clinical parameter, disease parameter or ionizing radiation treatment parameter determined in step (b) in a mathematical function to obtain an end-value.

2.  The *in vitro* method according to claim 1, wherein said at least one biochemical marker is radiation induced CD8+ T-lymphocytes apoptosis.

3.  The *in vitro* method according to claim 1 or 2, wherein the T stage of the tumor is also determined in step c) and a value associated to it combined in the function in step c).

4.  The *in vitro* method according to any one of claims 1 to 3, wherein step b) comprising the determination of at least another clinical marker, selected from the group consisting in the presence or absence of concomitant and/or adjuvant hormonotherapy, tobacco smoking habit, presence of gastrointestinal toxicity, dosage of PSA (Prostate Specific Antigen), presence of at least one comorbidity, tumor N stage and age of the patient, and wherein a value associated with said at least another clinical marker is combined in the function of c).

5.  The *in vitro* method according to claim 4, wherein the comorbidity is selected from the group consisting of presence or diabetes and high blood pressure associated with intake of anticoagulant drugs.

6.  The *in vitro* method according to anyone of claims 1 to 5, wherein the end value is compared with a reference end-value, thereby determining if the subject present a high or low risk to develop radiation-induced side-effects .

7.  The *in vitro* method according to any one of claims 1 to 6, wherein said mathematical function is a multivariate analysis using binary logistic regression, a multiple linear regression or any time dependent regression.

8. The *in vitro* method according to any one of claims 1 to 7, wherein said mathematical function is a COX proportional hazard regression model.

9. The *in vitro* method according to anyone of claims 1 to 8, wherein the sample of a) is a blood sample.

10. The *in vitro* method according to anyone of claims 1 to 9, wherein said radiation-induced side effect occurs within 24 months after the end of the ionizing radiation.

11. The *in vitro* method according to anyone of claims 1 to 10 wherein said radiation-induced side effect is selected from urinary toxicities, gastrointestinal toxicities, sexual toxicities and pelvic neuropathy.

12. The *in vitro* method according to anyone of claims 1 to 11, wherein said method is computerized.

13. A microprocessor comprising a software for implementing the *in vitro* method according to anyone of claims 1 to 12.

14. A method for treating a patient having prostate cancer, comprising:

a) performing the method according to any one of claims 1 to 12, and
b) treating the patient with ionizing radiation, if the end result indicates that the subject has no or a low risk of developing side effects after ionizing radiation,
c) providing another treatment or adapting the ionizing radiation treatment if the patient doesn't have no or a low risk of developing side effects after ionizing radiation.

15. A method for obtaining a mathematical function that can be used in an *in vitro* non-invasive prognosis test for assessing the risk of developing side effects after ionizing radiation in a subject suffering from prostate cancer, comprising:

a) classifying subjects of a cohort of patients into different groups according to the presence of side effects after ionizing radiation treatment
b) providing the value at least one biochemical marker in a sample of each subjects, wherein said at least one biochemical marker is radiation induced T-lymphocytes apoptosis (RILA) measured before ionizing radiation treatment; and
c) determining the presence or absence of at least one clinical parameter, disease parameter from each patient, wherein said at least one clinical parameter, disease parameter comprises presence of urinary toxicity prior to ionizing radiation treatment
d) optionally determining the presence of at least one other marker selected from the group consisting in the presence or absence of concomitant and/or adjuvant hormonotherapy, the tobacco smoking habit, the presence of gastrointestinal toxicity, the dosage of PSA (Prostate Specific Antigen), the presence of at least one comorbidity (in particular diabetes), the tumor T stage, the tumor N stage and the age of the patient, before ionizing radiation treatment
e) identifying by unidimensional analysis, the markers measured in step d) for which the measured value differs significantly between the group of subjects in which side effects after ionizing radiation have developed and the group of subjects in which side effects after ionizing radiation have not occurred, thereby obtaining independent factors
f) obtaining the mathematical function by combination of the factor measured in b), the marker determined in c) and the identified independent factors identified in e).

Europäisches
Patentamt

European
Patent Office

Office européen
des brevets

# EUROPEAN SEARCH REPORT

Application Number

EP 19 30 5262

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | FORO PALMIRA ET AL: "Relationship Between Radiation-Induced Apoptosis of T Lymphocytes and Chronic Toxicity in Patients With Prostate Cancer Treated by Radiation Therapy: A Prospective Study", INTERNATIONAL JOURNAL OF RADIATION: ONCOLOGY BIOLOGY PHYSICS, PERGAMON PRESS, USA, vol. 88, no. 5, 22 March 2014 (2014-03-22) , pages 1057-1063, XP028837765, ISSN: 0360-3016, DOI: 10.1016/J.IJROBP.2014.01.002 * the whole document * * In particular: Title; Abstract; Materials and Methods; Tables 1-4; Figures 1-2 * | 1-15 | INV. G01N33/50 |
| X | GUILLAUME VOGIN ET AL: "Absence of correlation between radiation-induced CD8 T-lymphocyte apoptosis and sequelae in patients with prostate cancer accidentally overexposed to radiation", ONCOTARGET, vol. 9, no. 66, 24 August 2018 (2018-08-24), pages 32680-32689, XP55612512, DOI: 10.18632/oncotarget.26001 * the whole document * * In particular: Title; Abstract; Page 32682-32684, bridging paragraph; Tables 1-3; Figures 1-4 * | 1-15 | |

TECHNICAL FIELDS
SEARCHED (IPC)

G01N

-/--

The present search report has been drawn up for all claims

| Place of search | Date of completion of the search | Examiner |
|---|---|---|
| The Hague | 16 August 2019 | C.F. Angioni |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

page 1 of 2

Europäisches
Patentamt

European
Patent Office

Office européen
des brevets

**EUROPEAN SEARCH REPORT**

Application Number

EP 19 30 5262

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | C. MIRJOLET ET AL: "RILA blood biomarker as a predictor of radiation-induced sarcoma in a matched cohort study", EBIOMEDICINE, vol. 41, 1 March 2019 (2019-03-01), pages 420-426, XP55612564, ISSN: 2352-3964, DOI: 10.1016/j.ebiom.2019.02.031 * the whole document * | 1-15 | |
| T | AZRIA D ET AL: "OC-0498 Results of the prospective trial evaluating radiation-induced lymphocyte apoptosis and prostate RT", RADIOTHERAPY AND ONCOLOGY, vol. 133, 1 April 2019 (2019-04-01), XP085699693, ISSN: 0167-8140, DOI: 10.1016/S0167-8140(19)30918-1 * the whole document * | | |

TECHNICAL FIELDS
SEARCHED      (IPC)

The present search report has been drawn up for all claims

| Place of search | Date of completion of the search | Examiner |
|---|---|---|
| The Hague | 16 August 2019 | C.F. Angioni |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2016008629 A

- WO 2018041960 A

**Non-patent literature cited in the description**

- **FORO et al.** *Int J Radiat Oncol Biol Phys.,* 01 April 2014, vol. 88 (5), 1057-63
- **DE LANGHE et al.** *Radiotherapy and Oncology,* 2013, vol. 106, S350

- **D'AMICO et al.** *JAMA,* 1998, vol. 280 (11), 969-74
- **AZRIA et al.** *Front Oncol.,* 27 April 2017, vol. 7, 83